# EUROPEAN PATENT SPECIFICATION

(11) **EP 2 649 037 B1**
(45) Date of publication and mention of the grant of the patent: **25.03.2020**
(21) Application number: 12708207.1
(22) Date of filing: 22.02.2012
(51) Int. Cl.: C07C 67/03, C07C 69/82, C08G 63/183, C08J 11/24, C08L 67/02, C08L 69/00

(54) **IMPACT MODIFIED POLY(BUTYLENE TEREPHTHALATE) ESTER COMPOSITIONS, METHODS OF MANUFACTURE, AND ARTICLES THEREOF**
ELASTIFIZIERTE POLY(BUTYLENTEREPHTHALAT-)ESTERZUSAMMENSETZUNG, HERSTELLUNGSVERFAHREN DAFÜR UND ARTIKEL DARAUS
COMPOSITIONS D'ESTER DE POLY(BUTYLENE TEREPHTHALATE) À IMPACT MODIFIÉ, PROCÉDÉ DE PRÉPARATION ET LEURS ARTICLES

(30) Priority: 22.02.2011 US 201113032121
(43) Date of publication of application: 16.10.2013
(73) Proprietor: SABIC Global Technologies B.V., 4612 PX Bergen op Zoom (NL)
(72) Inventor: BELL, Philip Wesley, Mount Vernon, Indiana 47620 (US); SHAH, Dhaval, Evansville, Indiana 47712 (US)
(74) Representative: Stiebe, Lars Magnus
(86) International application number: PCT/US2012/026048
(87) International publication number: WO 2012/116026

(56) References cited:
- EP-A1- 1 344 765
- WO-A1-95/11218
- WO-A1-2007/089600
- US-A- 5 391 263
- US-A1- 2010 168 317

## Description

### BACKGROUND

This disclosure relates to poly(butylene terephthalate) compositions, methods of manufacture, and articles thereof, wherein the poly(butylene terephthalate) is manufactured in part from recycled or scrap polyesters.

Thermoplastic polyesters such as poly(butyleneterephthalate) (PBT) are readily molded into useful articles, and articles comprising PBT have valuable characteristics including strength, toughness, high gloss, and solvent resistance. PBT therefore has utility in a wide range of applications, including automotive parts, electric appliances, and electronic devices. PBT is especially useful when blended with other components such as impact modifiers and polycarbonates.

WO 95/11218 A1 discloses a method for separating and removing impurities such as cyclohexane dimethanol and other additives from their mixture with dimethylterephthalate, formed during a depolymerization process. The separation and removal of cyclohexane dimethanol is accomplished via ether melt or solution crystallization of the dimethylterephthalate following excess methanol removal (via distillation) of the methanolysis reactor products.

US application 5,391,264 describes a method to separate ethylene glycol and diethylene glycol from dimethyl terephthalate accomplished by distillation using methyl benzoate or the methyl ester of p-toluic acid as an azeotropic agent.

EP 1344765 describes a dimethyl terephthalate composition containing 0.001 to 200 ppm of methyl 4-(1,3-dioxolan-2-yl)benzoate and 0 to 1 ppm of dimethyl hydroxyterephthalate that exhibits improved properties as a material for producing polyester; and the method to produce said composition.

US application 2010/168317 describes a composition comprising from 5 to 90 wt. % of a modified poly(butylene terephthalate) copolymer, at least 1 wt. % of an impact modifier component, and optional ly, from 0 to 5 wt. % of an additive and its method of manufacture.

WO 2007/089600 relates to a process for making modified polybutylene terephthalate random copolymers from a polyethylene terephthalate component.

Many manufacturers and consumers prefer to use more ecologically acceptable PBT. One method of making PBT more ecologically acceptable is to use recycled or scrap material in its manufacture. A particular challenge, however, in using recycle or scrap material is attaining properties comparable to the PBT derived from virgin material. There accordingly remains a need in the art for PBT derived at least in part from recycle or scrap material wherein the critical properties of the PBT are comparable to PBT derived from virgin material, and for compositions containing such PBT.

### BRIEF SUMMARY OF THE INVENTION

The present invention refers to a poly(butylene terephthalate) composition comprising a poly(butylene terephthalate) copolymer reaction product of a dimethyl terephthalate residual composition with 1,4-butanediol, wherein the dimethyl terephthalate composition comprises:
a. dimethyl terephthalate derived from a terephthalic-containing polyester homopolymer, terephthalic-containing polyester copolymer, or a combination thereof; and
b. from more than 0 to less than 4.2 wt.% of a residual component selected from dimethyl isophthalate, cyclohexanedimethanol, diethylene glycol, tri ethyl ene glycol, or a combination thereof.

In one embodiment, a poly(butylene terephthalate) composition comprises: (a) a poly(butylene terephthalate) copolymer reaction product of the dimethyl terephthalate residual composition as disclosed above with 1,4-butanediol, wherein the dimethyl terephthalate residual composition comprises from more than 0 to less than 4.2 wt.% of reaction residues of the residual component selected from dimethyl isophthalate, cyclohexane di methanol, diethylene glycol, tri ethyl ene glycol, or a combi nation thereof; (b) a polycarbonate; and (c) an impact modifier; wherein the polybutylene terephthalate composition has a heat deflection temperature ranging from 87.3 C to 101.7 C, when measured at 0.455 MPa in accordance with AST M method D648.

In another embodiment, a poly(butylene terephthalate) composition comprises
from 30 to 90 wt.% of the poly(butylene terephthalate) copolymer reaction product of the dimethyl terephthalate residual composition as disclosed above with 1,4-butanediol, wherein the dimethyl terephthalate residual composition comprises from more than 0 to less than 4.2 wt.% of reaction residues of the residual component selected from dimethyl isophthalate, cyclohexane dimethanol, diethylene glycol, tri ethylene glycol, or a combination thereof; from 10 to 80 wt% of a polycarbonate; from 0 to 25 wt.% of an impact modifier; from more than 0 to 2 wt.% of an antioxidant; from more than 0 to 2 wt.% of a mold release agent; from more than 0 to 2 wt.% of a quencher; and from more than 0 to 2 wt.% of an ultraviolet light stabilizer.

A method for the manufacture of the poly(butylene terephthalate) compositions comprises blending the components of the composition.

Further disclosed are articles comprising the poly(butylene terephthalate) compositions. Methods of manufacturing an article comprise shaping by extrusion, calendaring, molding, or injection molding the poly(butylene terephthalate) composition.

The above descri bed and other features are exempl ifi ed by the following detailed description.

### BRIEF DESCRIPTION OF THE DRAWINGS

Figure 1 shows melting point as a function of the percentage of total impurities in the PBT made in Examples 1-10.
Figure 2 shows crystallization temperature as a function of the percentage of total impurities in the PBT made in E xampl es 1-10.
Figure 3 shows heat deflection temperature with a loading stress of 0.455 MPa as a function of the percentage of total i mpuriti es present in the di methyl terephthalate used to make the PBT used in the stabilized PBT made in Examples 11-20.
Figure 4 shows heat deflection temperature with a loading stress of 1.82 MPa as a function of the percentage of total i mpuriti es present in the di methyl terephthalate used to make the PBT used in the stabilized PBT made in Examples 11-20.
Figure 5 shows mold shrinkage measured in the parallel direction as a function of the percentage of total impurities present in the dimethyl terephthalate used to make the PBT used in the stabilized PBT made in Examples 11-20.
Figure 6 shows mold shrinkage measured in the perpendicular direction as a function of the percentage of total impurities present in the dimethyl terephthalate used to make the PBT used in the stabilized PBT made in Examples 11-20.
Figure 7 shows tensile elongation at break as a function of the percentage of total impurities present in the dimethyl terephthalate used to make the PBT used in the stabilized PBT made in Examples 11-20.
Figure 8 shows heat deflection temperature with a loading stress of 0.455 MPa as a function of the percentage of total impurities present in the dimethyl terephthalate used to make the PBT used in the flame retardant composition with a very high PBT content made in Examples 21-30.
Figure 9 shows heat deflection temperature with a loading stress of 1.82 M Pa as a function of the percentage of total impurities present in the dimethyl terephthalate used to make the PBT used in the flame retardant compositi on with a very high PBT content made in Examples 21-30.
Figure 10 shows total energy measured by the biaxial impact test as a function of the percentage of total impurities present in the dimethyl terephthalate used to make the PBT used in the flame retardant composition with a very high PBT content made in Examples 21-30.
Figure 11 shows heat deflection temperature with a loading stress of 0.455 MPa as a function of the percentage of total impurities present in the dimethyl terephthalate used to make the PBT used in the impact modified composition with a polycarbonate/ PBT blend using a high PBT content made in Examples 31-40.
Figure 12 shows heat deflection temperature with a loading stress of 1.82 MPa as a function of the percentage of total impurities present in the dimethyl terephthalate used to make the PBT used in the impact modifi ed composition with a polycarbonate/ PBT blend using a high PBT content made in Examples 31-40.
Figure 13 shows modulus of elasticity as a function of the percentage of total impurities present in the dimethyl terephthalate used to make the PBT used in the impact modified composition with a polycarbonate / PBT blend using a high PBT content made in Examples 31-40.

### DETAILED DESCRIPTION OF THE INVENTION

Our invention is based on the discovery that it is now possible to make an impact-modified, polycarbonate-poly(butylene terephthalate) (PBT) composition where the PBT is derived at least in part from recycle or scrap polyesters. The impact-modified compositions having a combination of desirable properties, including heat deflection temperature and tensile elongation. In particular, the PBT is manufactured using a dialkyl terepthalate residual composition that is derived from a terephthalic acid-based polyester. It has been discovered that careful selection and regulation of certain amounts and/or types of impurities in the residual composition allows the manufacture of PBT from scrap or recycle polyesters that has properties comparable to those of PBT derived from virgin monomers.

As used herein, the terms 'recycle_ or 'scrap_ are not intended to limit the source of the terephthalic acid-based polyester, as any terephthalic acid-based polyester can be used regardless of its source.

As used herein the singular forms 'a,_ 'an,_ and 'the_ include plural referents. The term 'combination_ is inclusive of blends, mixtures, alloys, reaction products, and the like. Unless defined otherwise, technical and scientific terms used herein have the same meaning as is commonly understood by one of skill. Compounds are described using standard nomenclature. The term "and a combination thereof_ includes the named component and/or other components not specifically named having essentially the same function.

Other than in the operating examples or where otherwise indicated, all numbers or expressions referring to quantities of ingredients, reaction conditions, and the like, used in the specification and claims are to be understood as modified in all instances by the term 'about._ Various numerical ranges are disclosed in this patent application. Because these ranges are continuous, they include every value between the minimum and maximum values. The endpoints of all ranges reciting the same characteristic or component are independently combinable and inclusive of the recited endpoint. Unless expressly indicated otherwise, the various numerical ranges specified in this application are approximations. The term 'from more than 0 to_ an amount means that the named component is present in some amount that is greater than 0, and up to and including the higher named amount.

The PBT composition comprises a PBT that has been manufactured using a terephthalic acid diester monomer derived from a terephthalic acid-containing homopolymer, a terephthalic-containing copolymer, or a combination thereof. For example, the PBT can be using a terephthalic acid diester monomer derived from a terephthalic acid-containing polyester, wherein the polyester can be a homopolymer, a copolymer, or a combination thereof. For convenience such polyester homopolymers, copolymers, and combination can be collectively referred to herein as a poly(hydrocarbylene terephthalate). Poly(hydrocarbyleneterephthalate)s may contain units derived from dicarboxylic acids in addition to the terephthalic acid, for example isophthalic acid, adipic acid, glutaric acid, azelaic acid, sebacic acid, fumaric acid, and the various isomers of cyclohexane dicarboxylic acid. The poly(hydrocarbyleneterephthalate)s can be a poly(alkyleneterephthalate), wherein the alkylene group comprises 2 to 18 carbon atoms. Examples of alkylene groups include ethylene, 1,2-butylene, 1,3-butylene, 1,4-butylene, trimethylene, pentylene, hexylene, cyclohexylene, 1,4-cyclohexylene, and 1,4-cyclohexanedi methyl ene. A combination comprising at least one of the foregoing alkylene groups can be present. Poly(arylene terephthalate)s can also be used, for example poly(naphthalene terephthalate)s based on naphthalene dicarboxylic acids, including the 2,6-, 1,4-, 1,5-, or 2,7-isomers but the 1,2-, 1,3-, 1,6-, 1,7-, 1,8-, 2,3-, 2,4-, 2,5-, and/or 2,8-isomers. The poly(hydrocarbylene terephthalate)s can also have a combination of alkylene and arylene groups. In a specific embodiment, the poly(hydrocarbylene terephthalate) is poly(ethylene terephthalate). As described above, other alkylene or arylene groups can be present, in addition to residues derived from other dicarboxylic acids.

The residual composition comprising the diester monomer derived from the poly(hydrocarbylene terephthalate) can comprise a (C₁₋₃)alkyl ester of terephthalic acid, and in particular a dimethyl terephthalate (DMT). The dimethyl terephthalate residual composition can be made by any suitable process, provided that the resulting dimethyl terephthalate residual composition includes more than 0 to less than 5.1 wt.% of a residual component selected from dimethyl isophthalate, cyclohexanedi methanol, diethylene glycol, triethylene glycol, or a combinations thereof. In an embodiment, our compositions can be made by a process that involves depolymerization of a polyester by, for example, treatment of bulk poly(ethylene terephthalate) with super-heated methanol vapor in the presence of a suitable transesterifi cation catalyst at atmospheric pressure, provided that the resulting dimethyl terephthalate residual composition includes more than 0 to less than 5.1 wt.% of a residual component selected from dimethyl isophthalate, cyclohexane dimethanol, diethylene glycol, triethylene glycol, or a combinations thereof. In another embodiment, poly(alkylene terephthalate, particularly poly(ethylene terephthalate), can alternatively be treated with steam at a temperature from about 200éC to about 450éC, where the steam-treated poly(ethylene terephthalate) is then reduced from a brittle solid product to a powder having a mean particles size of from about 0.0005 to 0.002 millimeters, after which the fine powder is atomized with a gaseous substance including inert gas and methanol vapor to form an aerosol. The aerosol is conducted through a reaction zone at a temperature of 250éC to 300éC in the presence of excess methanol vapors to produce DMT, provided that the resulting dimethyl terephthalate residual composition includes more than 0 to less than 5.1 wt.% of a residual component selected from dimethyl isophthalate, cyclohexane dimethanol, diethylene glycol, tri ethylene glycol, or a combinations thereof. Alternatively, poly(ethylene terephthalate) waste can be subdivided into dimensions between 4 and 35 mesh and treated at a temperature of 100éC to 300éC in the presence of methanol and acid catalysts to produce DMT, provided that the resulting dimethyl terephthalate residual composition includes more than 0 to less than 5.1 wt.% of a residual component selected from dimethyl isophthalate, cyclohexane dimethanol, diethylene glycol, tri ethylene glycol, or a combinations thereof. Scrap polyester can be dissolved in oligomers of ethylene glycol and terephthalate acid or dimethyl terephthalate, followed by passing super-heated methanol through the solution, where the ethylene glycol and dimethyl terephthalate are subsequently recovered overhead. In still another embodiment, our composition can be made by processes that use methanol vapor under pressurized conditions, provided that the resulting dimethyl terephthalate residual composition includes more than 0 to less than 5.1 wt.% of a residual component selected from dimethyl isophthalate, cyclohexane dimethanol, diethylene glycol, triethylene glycol, or a combinations thereof. The temperature at which the process to make the residual composition is practiced can vary. In an embodiment, when a process that uses methanol under pressure is used in conjunction with a transesterification catalyst, the process is practiced at a temperature ranging from 120-200 eC, or higher than 200 éC.

The monomers produced from these processes can be purified, for example by techniques such as distillation, crystallization, and filtration. However, the cost of the purification steps can render the recovered monomers more expensive than virgin raw materials. Surprisingly, it has been found by the inventors hereof that only very specific levels of impurities can be present in the (C₁₋₃)alkyl terephthalic esters, in particular DMT, used to produce PBT, yielding PBT that has properties comparable to PBT produced from virgin monomers. Without being bound by theory, it is believed that at least a portion of the impurities are incorporated into the PBT backbone, resulting in a copolymer with altered properties.

Thus, a di(C₁₋₃)alkyl terephthalic ester residual composition, and a DMT residual composition in particular derived from a terephthalic-containing polyester homopolymer, terephthalic-containing copolymer, or a combination thereof and containing from more than 0 to less than 4.2 wt.% of a residual component selected from di methyl isophthalate, cyclohexane dimethanol, diethylene glycol, triethylene glycol, and combinations thereof can be successfully used in the manufacture of PBT that can be used in an impact-modified polycarbonate-PBT composition having commercially valuable properties.

Methods for the manufacture of PBT from a di(C₁₋₃)alkyl terephthalic esters are known, and can be used to manufacture PBT from a di(C₁₋₃)alkyl terephthalic ester residual compositions, and in particular the DMT residual compositions, and 1,4-butanediol. For example, polyesters can be obtained by interfacial polymerization, melt-process condensation, or solution phase condensation in the presence of an acid catalyst. The catalyst facilitates the transesterification reaction, and can be selected from antimony compounds, tin compounds, titanium compounds, combinations thereof as well as many other metal catalysts and combinations of metal catalysts that have been disclosed in the literature. The amount of catalyst required to obtain an acceptable polymerization rate at the desired polymerization temperature will vary, and can be determined by experimentation. The catalyst amount can be 1 to 5000 ppm, or more. It is possible to prepare a branched polyester in which a branching agent, for example, a glycol having three or more hydroxyl groups or a trifunctional or multifunctional carboxylic acid has been incorporated. Furthermore, it is sometime desirable to have various concentrations of acid and hydroxyl end groups on the polyester, depending on the ultimate end use of the composition.

In general, the PBT manufactured from 1,4-butanediol and the DMT residue from more than 0 to less than 5.1 wt.%, specifically less than 4.2 wt% of a residual component selected from dimethyl isophthalate, cyclohexanedimethanol, diethylene glycol, triethylene glycol, and combinations thereof can have an intrinsic viscosity of 0.4 to 2.0 deciliters per gram (dL/g), specifically 0.5 to 1.5 dL/g, more specifically 0.6 to 1.2 dL/g, as measured in a 60:40 by weight phenol/1,1,2,2-tetrachloroethane mixture at 23éC. The PBT can have a weight average molecular weight of 10,000 to 200,000 Daltons, specifically 50,000 to 150,000 Daltons as measured by gel permeation chromatography (GPC). The polyester component can also comprise a mixture of different batches of PBT prepared under different process conditions in order to achieve different intrinsic viscosities and/or weight average molecular weights.

In a specific embodiment, the PBT reaction product of 1,4-butanediol and the DMT residue having from more than 0 to less than 5.1 wt.%, specifically 4.2 wt.%, of a residual component selected from dimethyl isophthalate, cyclohexanedimethanol, diethylene glycol, triethylene glycol, and combinations thereof can have a melting temperature that is within 1.5· C of the melting temperature of the same PBT that is the reaction product of virgin dimethyl terepthalate and 1,4-butanediol.

The PBT reaction product of 1,4-butanediol and the DMT residue having from more than 0 to less than 5.1 wt.%, specifically 4.2 wt.% of a residual component selected from dimethyl isophthalate, cyclohexane dimethanol, diethylene glycol, triethylene glycol, and combinations thereof butanediol can have a crystallization temperature that is within 7.4·C of the crystallization temperature of the same PBT that is the reaction product of virgin dimethyl terepthalate.

The PBT reaction product of the DMT residue and 1,4-butanediol can be used a component in an impact-modified polycarbonate-PBT composition having a high percentage of PBT. The impact-modified polycarbonate-PBT composition can further contain a flame retardant.

Polycarbonates for use in the impact-modified compositions are known in the art, a compositions having repeating structural carbonate units of the formula (1): 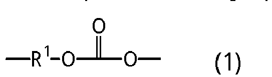 in which at least 60 percent of the total number of R¹ groups contain aromatic organic groups and the balance thereof are aliphatic, alicyclic, or aromatic groups. In an embodiment, each R¹ is a C₆₋₃₀ aromatic group, that is, contains at least one aromatic moiety. R¹ can be derived from a dihydroxy compound of the formula HO-R¹-OH, in particular a dihydroxy compound of formula (2):

HO⁻A¹⁻Y¹⁻A²⁻OH (2)

wherein each of A¹ and A² is a monocyclic divalent aromatic group and Y¹ is a single bond or a bridging group having one or more atoms that separate A¹ from A². In an exemplary embodiment, one atom separates A¹ from A². Specifically, each R¹ can be derived from a dihydroxy aromatic compound of formula (3) 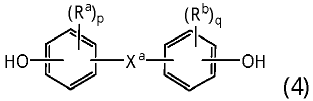 wherein R^{a} and R^{b} each represent a halogen or C₁₋₁₂ alkyl group and can be the same or different; and p and q are each independently integers of 0 to 4. Also in formula (6), X^{a} represents a bridging group connecting the two hydroxy-substituted aromatic groups, where the bridging group and the hydroxy substituent of each C₆ arylene group are disposed ortho, meta, or para (specifically para) to each other on the C₆ arylene group. In an embodiment, the bridging group X^{a} is single bond, -O-, -S-, -S(O)-, -S(O)₂-, -C(O)-, or a C₁₋₁₈ organic group. The C₁₋₁₈ organic bridging group can be cyclic or acyclic, aromatic or non-aromatic, and can further comprise heteroatoms such as halogens, oxygen, nitrogen, sulfur, silicon, or phosphorous. The C₁₋₁₈ organic bridging group can be disposed such that the C₆ arylene groups connected thereto are each connected to a common alkylidene carbon or to different carbons of the C₁₋₁₈ organic bridging group. In one embodiment, p and q is each 1, and R^{a} and R^{b} are each a C₁₋₃ alkyl group, specifically methyl, disposed meta to the hydroxy group on each arylene group.

In an embodi ment, X^{a} is a substituted or unsubstituted C₃₋₁₈ cycloalkylidene, a C₁₋₂₅ alkylidene of formula ⁻C(R^{c})(R^{d})⁻ wherein R^{c} and R^{d} are each independently hydrogen, C₁₋₁₂ alkyl, C₁₋₁₂ cycloalkyl, C₇₋₁₂ arylalkyl, C₁₋₁₂ heteroalkyl, or cyclic C₇₋₁₂ heteroarylalkyl, or a group of the formula ⁻C(=R^{e})⁻ wherein R^{e} is a divalent C₁₋₁₂ hydrocarbon group. Exemplary groups of this type include methylene, cyclohexylmethylene, ethyl idene, neopentyl idene, and isopropyl idene, as well as 2-[2.2.1]-bicycloheptylidene, cyclohexylidene, cyclopentylidene, cyclododecylidene, and adamantylidene.

Other useful aromatic di hydroxy compounds of the formula HO-R¹-OH include compounds of formula (4) 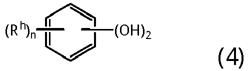 herein each R^{h} is independently a halogen atom, a C₁₋₁₀ hydrocarbyl such as a C₁₋₁₀ alkyl group, a halogen-substituted C₁₋₁₀ alkyl group, a C₆₋₁₀ aryl group, or a halogen-substituted C_{6- 10} aryl group, and n is 0 to 4. The halogen is usually bromine.

Some illustrative examples of specific aromatic dihydroxy compounds include the following: 4,4'-dihydroxybiphenyl, 1,6-dihydroxynaphthalene, 2,6-dihydroxynaphthalene, bis(4-hydroxyphenyl) methane, bis(4-hydroxyphenyl)diphenylmethane, bis(4-hydroxyphenyl)-1-naphthyl methane, 1,2-bis(4-hydroxyphenyl)ethane, 1,1-bis(4-hydroxyphenyl)-1-phenylethane, 2-(4-hydroxyphenyl)-2-(3-hydroxyphenyl) propane, bis(4-hydroxyphenyl) phenyl methane, 2,2-bis(4-hydroxy-3-bromophenyl)propane, 1,1-bis (hydroxyphenyl)cyclopentane, 1,1-bis(4-hydroxyphenyl)cyclohexane, 1,1-bis(4-hydroxyphenyl)isobutene, 1,1-bis(4-hydroxyphenyl)cyclododecane, trans-2,3-bis(4-hydroxyphenyl)-2-butene, 2,2-bis(4-hydroxyphenyl)adamantine, alpha, alpha'-bis(4-hydroxyphenyl)toluene, bis(4-hydroxyphenyl)acetonitrile, 2,2-bis(3-methyl-4-hydroxyphenyl)propane, 2,2-bis(3-ethyl-4-hydroxyphenyl)propane, 2,2-bis(3-n-propyl-4-hydroxyphenyl)propane, 2,2-bis(3-isopropyl-4-hydroxyphenyl) propane, 2,2-bis(3-sec-butyl-4-hydroxyphenyl)propane, 2,2-bis(3-t-butyl-4-hydroxyphenyl) propane, 2,2-bis(3-cyclohexyl-4-hydroxyphenyl)propane, 2,2-bis(3-allyl-4-hydroxyphenyl)propane, 2,2-bis(3-methoxy-4-hydroxyphenyl)propane, 2,2-bis(4-hydroxyphenyl) hexafluoropropane, 1,1-dichloro-2,2-bis(4-hydroxyphenyl) ethyl ene, 1,1-dibromo-2,2-bis(4-hydroxyphenyl)ethylene, 1,1-dichloro-2,2-bis(5-phenoxy-4-hydroxyphenyl)ethylene, 4,4'-dihydroxybenzophenone, 3,3-bis(4-hydroxyphenyl)-2-butanone, 1,6-bis(4-hydroxyphenyl)-1,6-hexanedione, ethylene glycol bis(4-hydroxyphenyl) ether, bis(4-hydroxyphenyl)ether, bis(4-hydroxyphenyl)sulfide, bis(4-hydroxyphenyl)sulfoxide, bis(4-hydroxyphenyl)sulfone, 9,9-bis(4-hydroxyphenyl)fluorine, 2,7-dihydroxypyrene, 6,6'-dihydroxy-3,3,3',3'-tetramethylspiro(bis)indane ("spirobiindane bisphenol"), 3,3-bis(4-hydroxyphenyl)phthalide, 2,6-dihydroxydibenzo-p-dioxin, 2,6-di hydroxythianthrene, 2,7-di hydroxyphenoxathi n, 2,7-dihydroxy-9,10-dimethyl phenazine, 3,6-dihydroxydibenzofuran, 3,6-dihydroxydibenzothiophene, and 2,7-dihydroxycarbazole, resorcinol, substituted resorcinol compounds such as 5-methyl resorcinol, 5-ethyl resorcinol, 5-propyl resorcinol, 5-butyl resorcinol, 5-t-butyl resorcinol, 5-phenyl resorcinol, 5-cumyl resorcinol, 2,4,5,6-tetrafluoro resorcinol, 2,4,5,6-tetrabromo resorcinol, or the like; catechol; hydroquinone; substituted hydroqui nones such as 2-methyl hydroquinone, 2-ethyl hydroquinone, 2-propyl hydroquinone, 2-butyl hydroquinone, 2-t-butyl hydroquinone, 2-phenyl hydroquinone, 2-cumyl hydroquinone, 2,3,5,6-tetramethyl hydroquinone, 2,3,5,6-tetra-t-butyl hydroquinone, 2,3,5,6-tetrafluoro hydroquinone, 2,3,5,6-tetrabromo hydroquinone, and the like, as well as combinations comprising at least one of the foregoing di hydroxy compounds.

Specific examples of bisphenol compounds include 1,1-bis(4-hydroxyphenyl) methane, 1,1-bis(4-hydroxyphenyl) ethane, 2,2-bis(4-hydroxyphenyl) propane (hereinafter "bisphenol A_ or 'BPA_), 2,2-bis(4-hydroxyphenyl) butane, 2,2-bis(4-hydroxyphenyl) octane, 1,1-bis(4-hydroxyphenyl) propane, 1,1-bis(4-hydroxyphenyl) n-butane, 2,2-bis(4hydroxy-1-methylphenyl) propane, 1,1-bis(4-hydroxy-t-butylphenyl) propane, 3,3-bis(4-hydroxyphenyl) phthalimidine, 2-phenyl-3,3-bis(4-hydroxyphenyl) phthalimidine (PPPBP), and 1,1-bis(4-hydroxy-3-methylphenyl)cyclohexane (DMBPC). Combinations comprising at least one of the foregoing dihydroxy compounds can also be used. In one specific embodiment, the polycarbonate is a linear homopolymer derived from bisphenol A, in which each of A¹ and A² is p-phenylene and Y¹ is isopropylidene in formula (4).

In a specific embodiment, the polycarbonate can be a linear homopolymer derived from bisphenol A, in which each of A¹ and A² is p-phenylene and Y¹ is isopropylidene. The polycarbonates generally can have an intrinsic viscosity, as determined in chloroform at 25· C, of 0.3 to 1.5 deciliters per gram (dl/g), specifically 0.45 to 1.0 dl/g. The polycarbonates can have a weight average molecular weight (Mw) of 10,000 to 100,000 g/mol, as measured by gel permeation chromatography (GPC) using a crosslinked styrene-divinyl benzene column, at a sample concentration of 1 milligram per milliliter, and as calibrated with polycarbonate standards.

In an embodi ment, the polycarbonate can have a melt volume flow rate (often abbreviated MV R) measures the rate of extrusion of a thermoplastics through an orifice at a prescribed temperature and load. Polycarbonates useful for the formation of articles can have an MVR, measured at 300éC under a load of 1.2 kg according to ASTM D1238-04 or ISO 1133, of 0.5 to 80 cubic centimeters per 10 minutes (cc/10 min). In a specific embodiment, where a polycarbonate is used in addition to the polyestercarbonate, the polycarbonate (or a combination of polycarbonates, i.e., a polycarbonate composition) can have an MVR measured at 300éC under a load of 1.2 kg accordi ng to A ST M D1238-04 or ISO 1133, of 5 to 35 cc/10 min, specifically 10 to 35 cc/10 min, and more specifically 25 to 35 cc/10 min.

The impact-modified compositions further comprise an impact modifier, which can be one of the known impact modifiers useful for polyesters, polycarbonates, or their blends. The impact modifier component is general ly a rubbery material, which when used in suitable amounts, imparts energy absorbing properties to the composition. Suitable rubbery impact modifiers include (a) methacrylate butadiene styrene rubbers, (b) acrylate rubbers, (c) acrylonitrile-styrene-acrylate rubbers, (d) high rubber graft acrylonitrile-butadiene-styrenes, (e) acrylate-olefin copolymers, (f) polyolefin modifiers, or (g) silicone-acrylic modifiers (e.g., METABLEN™ made by Mitsubishi Rayon).

More particularly, the impact modifier can include an acrylonitrile-butadiene-styrene (A BS) polymer of the high rubber graft impact modifier. Rubber modified monovinylidene aromatic resins comprising (a) a rubber modified monovinylidene aromatic graft copolymer and (b) an ungrafted rigid copolymer, are generally prepared by graft polymerization of a mixture of a monovinyl idene aromatic monomer and one or more comonomers in the presence of one or more rubbery polymeric substrates. Depending on the amount of rubber present, a separate matrix or continuous rigid phase of ungrafted rigid (co)polymer may be simultaneously obtained along with the rubber modified monovinylidene aromatic graft polymer. The resins may also be produced by blending a rigid monovinylidene aromatic copolymer with one or more rubber modified monovinylidene aromatic graft copolymers.

Typically, the rubber modified resins comprise the rubber modified graft copolymer at a level of from 5 to 100 percent by weight based on the total weight of the resin, more preferably from 10 to 95 percent by weight thereof, more preferably 20 to 90 percent by weight thereof, and most preferably from 15 to 85 percent by weight thereof; and the rubber modified resin comprises the ungrafted rigid polymer at a level of from 0 to 95 percent by weight based on the total weight of the resin, more preferably from 5 to 90 percent by weight thereof, more preferably from 10 to 80 percent by weight thereof and most preferably from 15 to 85 percent by weight thereof. Higher levels of rubber are preferred.

Especially preferred are acrylonitrile-butadiene-styrene copolymers having greater than 30% by weight rubbery polymeric substrate, preferable greater than about 45% by weight rubbery polymeric substrate. The most preferred rubbery substances comprise polybutadiene or styrene-butadiene copolymer. A Iso preferred are high rubber graft acrylonitrile-butadiene-styrene copolymer. The phrase "high rubber graft" refers generally to graft copolymer resins wherein at least about 30% by weight, preferably at least about 45% by weight of the rigid polymeric phase is chemically bound or grafted to the rubbery polymeric phase. Suitable A BS-type high rubber graft copolymers are commercially available from, for example, under the trademark BLENDEX ÷ resin 336 or 338. One preferred high rubber graft is CYCOLAC ÷ C874202 resin of General Electric Company, Advanced Materials. A process for producing graft copolymer resins is set forth in U.S. Pat. No. 6,384,129 to Lowry.

Other typical impact modifiers are the following materials, or blends of two or more of these materials: (1) Paraloid EX L3300, which is butyl acrylate-methacrylate core-shell rubber; (2) ASA-H RG, which is acrylonitrile-styrene-butyl acrylate copolymer; (3) AES, which is acrylonitrile-styrene-EPDM copolymer, where EPDM is ethylene-propylene non conjugated diene elastomer, (4) LOTADER AX 8900, which is ethylene-methacrylate-glycidyl methacrylate copolymer with methacrylate content of about 8%. The content of impact modifier is preferable less than 40% by weight, more preferable less than 30 percent, and most preferable less than 20 percent

Core-shell copolymers, method of making core-shell copolymers and the use of core-shell copolymers as impact modifiers in combination with polycarbonate are described in U.S. Pat. Nos. 3,864,428 and 4,264,487. Suitable core-shell copolymers are those that include a rubbery "core" that has a glass transition temperature ("Tg") below about 10éC. and that comprises repeating units derived from one or more monoethylenically unsaturated monomers such as, e.g., acrylate monomers, such as butyl acrylate, and conjugated diene monomers, e.g., butadiene and a rigid "shell" that has a Tg of greater than or equal to about 10éC and that has repeating units derived from a monoethylenically unsaturated monomer.

The impact-modified compositions comprise from 30 to 90 wt.% of the polybutylene terepthalate copolymer reaction product of the dimethyl terephthalate residual composition of claim 1 and butane diol; from 10 to 80 wt.% of the polycarbonate; and from more than 0 to 25 wt.% of the impact modifier, based on the total weight of the impact-modified composition. In a specific embodiment, the impact-modified compositions comprise from 30 to 90 wt.% of the polybutylene terepthalate copolymer reaction product of the dimethyl terephthalate residual composition of claim 1 and butane diol; from 10 to 69 wt.% of the polycarbonate; and from 1 to 22 wt.% of the impact modifier, based on the total weight of the impact-modified composition. In another specific embodiment, the impact-modified compositions comprise from 40 to 80 wt.% of the polybutylene terepthalate copolymer reaction product of the dimethyl terephthalate residual composition of claim 1 and butane diol; from 10 to 59 wt.% of the polycarbonate; and from 1 to 15 wt.% of the impact modifier, based on the total weight of the impact-modified composition.

With the proviso that desired properties, such as mold shrinkage, tensile elongation, heat deflection temperature, and the like are not adversely affected, the thermoplastic compositions comprising PBT manufactured from the di(C₁₋₃)alkyl terephthalic ester residue, specifically the DMT residue, can optionally further comprise other known additives used in thermoplastic polyester compositions such as non-reinforcing fillers, stabilizers such as antioxidants, thermal stabilizers, radiation stabilizers, and ultraviolet light absorbing additives, as well as mold release agents, plasticizers, quenchers, lubricants, antistatic agents, dyes, pigments, laser marking additives, and processing aids. A combination comprising one or more of the foregoing or other additives can be used. When used, the additives are each generally present in an amount of 0.01 to 5 wt.%, specifically 0.05 to 2 wt.%. A specific example of an additive combination is an antioxidant such as a hindered phenol stabilizer, a quencher, an ultraviolet light stabilizer, and a waxy mold release agent such as pentaerythritol tetrastearate, each present in an amount from more than 0, specifically 0.1 to 2 wt.%, based on the total weight of the impact-modified composition.

One or more flame retardants can also be present in the impact modified compositions, selected from sulfonated salts, brominated compounds, antimony flame retardants, phosphorus-containing flame retardants, nitrogen-containing flame retardants, phosphinate salts, and combinations thereof.

Inorganic flame retardants can be used, for example salts of C₂₋₁₆ alkyl sulfonate salts such as potassium perfluorobutane sulfonate (Rimar salt), potassium perfluorooctane sulfonate, tetraethyl ammonium perfluorohexane sulfonate, and potassium diphenylsulfone sulfonate, and the like; salts formed by reacting for example an alkali metal or alkaline earth metal (for example lithium, sodium, potassium, magnesium, calcium and barium salts) and an inorganic acid complex salt, for example, an oxo-anion, such as alkali metal and alkaline-earth metal salts of carbonic acid, such as Na₂CO₃, K₂CO₃, MgCO₃, CaCO₃, and BaCO₃ orfluoro-anion complexes such as Li₃AlF₆, BaSiF₆, KBF₄, K₃AlF₆, KAlF₄, K₂SiF₆, and/or Na₃AlF₆ or the like. When present, inorganic flame retardant salts can be present in amounts of 0.1 to 5 weight percent, based on the total weight of the composition.

Brominated organic compounds contain bromi ne atoms directly bonded to an organic moiety, for example an aromatic or cycloaliphatic ring, for example compounds derived from brominated Bisphenol A, tetrabromophthalic anhydride, and the like, and pentabromocyclohexane, pentabromochlorocyclohexane, hexabromocyclohexane, 1,2-dibromo-4-(1,2-dibromoethyl)-cyclohexane, tetrabromocyclooctane, hexabromocyclooctane, hexabromocyclododecane, chloroparaffins, and analogous bromine- or chlorine- containing aliphatic or cycloaliphatic compounds. Brominated polycarbonates include copolycarbonates prepared from brominated and unbrominated dihydroxy compounds. The ratio of brominated units to unbrominated units in the copolycarbonate is chosen to provide an amount of bromine of about 1 to about 45 weight percent, based on the total weight of the copolycarbonate. An exemplary brominated polycarbonate is a copolycarbonate comprising structural units derived from bisphenol A and tetrabromobisphenol A. Brominated carbonate oligomers can be used, which comprise about 1 to about 80 weight percent bromine based on the total weight of the oligomer, including for example those available from Great Lakes Chemical under the trade name GREAT LAKES BC-52, GREAT LAKES BC-52 HP, GREAT LAKES BC-58, and GREAT LAKES PE-68.

Organic flame retardants that can be used include various phosphorus-containing compounds, in particular aromatic phosphorus-containing compounds and brominated or chlorinated organic compounds. Organic compounds containing phosphorus-nitrogen bonds can be used, as well compounds such as phosphonate, phosphinate, phosphite, phosphine oxide, and phosphate esters can be used, for example triethyl phosphate, triphenyl phosphate, tris(dichloropropyl) phosphate; tris(2-chloroethyl)phosphate; tris(dibromopropyl) phosphate; tris(bromochloropropyl)phosphate; and the like.

The impact-modified composition can include a flame retarding quantity of one or a mixture of nitrogen-containing flame retardants such as triazines, guanidines, cyanurates, and isocyanurates. Exemplary triazines have the formula: 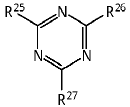 wherein R²⁵, R²⁶, and R²⁷are independently C₁-C₁₂ alkyl, C₁-C₁₂ alkoxy, C₆-C₁₂ aryl, amino, C₁-C₁₂ alkyl-substituted amino, or hydrogen. Highly preferred triazines include 2,4,6-triamine-1,3, 5-triazine(melamine, CAS Reg. No. 108-78-1), melamine derivatives, melam, melem, melon, ammeline(CAS Reg. No. 645-92-1), ammelide(CAS Reg. No. 645-93-2), 2-ureidomelamine, acetoguanamine(CAS Reg. No. 542-02-9), benzoguanamine(CAS Reg. No. 91-76-9), and the like. Salts/adducts of these compounds with boric acid or phosphoric acid can be used in the composition. Examples include melamine pyrophosphate and melamine polyphosphate (CAS Reg. No. 218768-84-4). Specific cyanurate/isocyanurate compounds include salts/adducts of the triazine compounds with cyanuric acid, such as melamine cyanurate and any mixtures of melamine salts. Specific guanidine compounds include guanidine; aminoguanidine; and the like; and their salts and adducts with boric acid, carbonic acid, phosphoric acid, nitric acid, sulfuric acid, and the like; and mixtures comprising at least one of the foregoing guanidine compounds. The nitrogen-containing flame retardant can be present in the impact-modified composition in an amount from 1 to 25 weight percent, based on the total weight of the composition. From 1 to 25 wt.% of a melamine polyphosphate, melamine cyanurate, melamine pyrophosphate, and/or melamine phosphate, based on the total weight of the compositi on can be used.

A flame retarding quantity of one or more phosphinic acid or diphosphinic acid salts can be used. The phosphinates and diphosphinates include those set forth in U.S. Patent No. 6,255,371 to Schosser et al. The phosphinates are of the formula (I)

[(R¹)(R²)(PO)-O]ₘ⁻M^{m+} (I),

the formula (II)

[(O-POR¹)(R³)(POR²-O)]²⁻ₙM^{m+}ₓ (II),

and/or polymers comprising formula (I) or (II), wherein R¹ and R² are identical or different and are H, Cₗ-C₆ linear or branched alkyl, or C₆-C₁₀ aryl; and R³ is C₁-C₁₀, linear or branched alkylene, C₆-C₁₀ arylene, C₇-C₁₁ alkylarylene, or C₇-C₁₁ arylalkylene; M is an alkaline earth metal, alkali metal, Al, Ti, Zn, Fe, or B; m is 1, 2, 3 or4; n is 1, 2, or3; and x is 1 or2.

In one embodiment R¹ and R² are the same and are C₁-C₆-linear or branched alkyl, or phenyl; R³ is C₁-C₁₀-linear or branched alkylene, C₆-C₁₀-arylene,-alkylarylene or-arylalkylene; M is magnesium, calcium, aluminum, zinc, or a combination thereof; m is 1, 2 or 3; n is 1, 2 or 3; and x is 1 or 2. In a specific embodiment M is aluminum. Specifically R¹ and R² can be H. When R¹ and R² are H, the phosphinate of formula (I) is a hypophosphite. Examples of hypophosphites are calcium hypophosphite, aluminum hypophosphite, and the like. Specific examples of phosphinate (I) are aluminum diethyl phosphinate (DEPAL) and zinc diethyl phosphinate (DEPZ N). The amount of phosphinate, diphosphinate, or polymers thereof, in the thermoplastic polyester composition can be from 1 to 35 wt.%, specifically from 5 to 20 wt%, and more specifically from 10 to 15 wt.%, based on the total weight of the impact-modified composition.

Adjuncts of flame retardants can also be used, for example an optional charring polymer. A charring polymer is a polymer that has not more than 85% weight loss at 400· C to 500· C upon heating under nitrogen using a thermogravimetric analysis (TGA) at a heating rate of 20· C per minute. Typical charring polymers include polyetherimides, poly(phenylene ether), poly(phenylenesulfide), polysulfones, polyethersulfones, poly(phenylenesulfide oxide) (PPSO), and polyphenolics (e.g., novolacs). The charring polymer can be present in an amount from 0.1 to 15 wt.%, based on the total weight of the thermoplastic polyester composition. In a specific embodiment, a polyetherimide is present, specifically an aromatic polyetherimide. When present, the polyetherimide can be in an amount from more than 0 to 25 wt. %, specifically 0.1 to 25 wt. %, even more specifically from 2 to 8 wt. %, each based on the total weight of the impact-modified composition.

One or more anti-dripping agents, which prevent or retard the resin from dripping while the resin is subjected to burning conditions, can optionally be present. Examples of such agents include silicone oils, silica (which also serves as a reinforcing filler), asbestos, and fibrillating-typefluoropolymers. Examples of fluoropolymers include fluorinated polyolefins such as poly(tetrafluoroethylene), tetrafluoroethylene/hexafluoropropyl ene copolymers, tetrafluoroethylene/ethylene copolymers, poly(vinylidene fluoride), poly(chlorotrifluoroethylene), and the like, and mixtures comprising at least one of the foregoing anti-dripping agents. A preferred anti-dripping agent is poly(tetrafluoroethylene) encapsulated by a poly(styrene-co-acrylonitrile) (SA N) copolymer. The anti-dripping agent is present in an amount of 0.01 to 5 wt.%, specifically from 0.05 to 2 wt.%, and more specifically 0.1 to 1 wt%, based on the total weight of the impact-modified composition.

The thermoplastic PBT composition can optionally comprise a reinforcing fiber. Any rigid reinforcing fiber can be used, for example, a glass fiber, a carbon fiber, a metal fiber, a ceramic fiber or whisker, and the like. The fibers can be chopped or continuous.

In some applications it can be desirable to treat the surface of the fiber with a chemical coupling agent to improve adhesion to the polyester in the thermoplastic polyester composition. Examples of useful coupling agents are alkoxy silanes and alkoxy zirconates. Amino, epoxy, amide, orthio functional alkoxy silanes are especially useful. Fiber coatings with high thermal stability are preferred to prevent decomposition of the coating, which could result in foaming or gas generation during processing at the high melt temperatures required to form the compositions into articles.

The fibers can have diameters of 6 to 30 micrometers. The reinforcing fibers can be provided in the form of monofilaments or multifilaments and can be used either alone or in combination with other types of fiber, through, for example, co-weaving or core/sheath, side-by-side, orange-type or matrix and fibril constructions, or by other methods known to one skilled in the art of fiber manufacture. Suitable cowoven structures include, for example, glass fiber-carbon fiber, carbon fiber-aromatic polyimide (aramid) fiber, and aromatic polyimide fiberglass fiber or the like. Reinforcing fibers may be supplied in the form of, for example, rovings, woven fibrous reinforcements, such as 0-90 degree fabrics or the like; non-woven fibrous reinforcements such as continuous strand mat, chopped strand mat, tissues, papers and felts or the like; or three-dimensional reinforcements such as braids.

In one embodiment, the reinforcing fiber is a glass fiber. An exemplary glass fiber is one that is relatively soda free, specifically fibrous glass filaments comprising lime-alumino-borosilicate glass, which is also known as 'E_glass. In a specific embodi ment the glass fiber has a diameter of 10 to 13 mi crometers. The glass fibers can be flat. E xempl ary flat glass fibers have a modul us of greater than or equal to 6,800 megaPascals (MPa), and can be chopped or continuous. The flat glass fiber can have various cross-sections, for example, trapezoidal, rectangular, or square. In preparing the molding compositions it is convenient to use a glass fiber in the form of chopped strands having an average length of from 0.1 mm to 10 mm, and having an average aspect ratio of 2 to 5. In articles molded from the compositions on the other hand shorter lengths will typically be encountered because during compounding considerable fragmentation can occur. When used, the glass fiber is present in an amount of 0.1 to 4.5 wt.%, more specifically 0.5 to 4 wt.%, and still more specifically 1 to 2.5 wt%, based on the weight of the thermoplastic PBT composition. When amounts less than 0.1 wt.% are used, there is no improvement in flexural modulus or tensile strength (tensile stress at yield). When amounts greater than or equal to 5 wt.% are used, the thermoplastic polyester compositi on will not meet the UL 94 V0 performance standard at a thickness of 0.8 mm and will not be sufficiently ductile.

In an embodiment, no non-fibrous inorganic filler is present. Alternatively, the thermoplastic PBT compositions can optionally comprise a non-fibrous inorganic filler, which can impart additional beneficial properties to the compositions such as thermal stability, increased density, stiffness, and/or texture. Non-fibrous inorganic fillers include, but are not limited to, alumina, amorphous silica, alumino silicates, mica, clay, talc, glass flake, glass microspheres, metal oxides such as titanium dioxide, zinc sulfide, ground quartz, and the like. The amount of non-fibrous filler can be in a range of between 0 wt.% and 50 wt.% based on the weight of the total composition.

The thermoplastic PBT compositions can optionally comprise both a reinforcing fiber and a platy filler. Combinations of glass fibers, carbon fibers or ceramic fibers with a platy filler, for example mica or flaked glass, can give enhanced properties. In general, the flat, plate-like filler has a length and width at least ten times greater than its thickness, where the thickness is from 1 to 1000 microns. Combinations of rigid fibrous fillers with flat, plate-like fillers can reduce warp of the molded article.

The thermoplastic compositions comprising PBT manufactured from the di(C₁₋₃)alkyl terephthalic ester residue, specifically the DMT residue, can be prepared by blending the components of the composition so that they are homogeneously dispersed in a continuous matrix of the polyester. A number of blending processes can be used. In an exemplary process, the PBT, other optional polymers, and/or other additives are mixed in an extrusion compounder and extruded to produce molding pellets. In another process, the components, including any reinforcing fibers and/or other additives, are mixed with the PBT by dry blending, fluxed on a mill and either comminuted or extruded and chopped. The components can also be mixed and directly molded, e.g. by injection or transfer molding. All of the components are dried as much as possible prior to blending. In addition, compounding is carried out with as short a residence time in the machine as possible and at the lowest possible temperature to minimize loss of components by decomposition or volatilization. The temperature is carefully controlled, and friction heat is utilized.

In one embodiment, the components are pre-compounded, pelletized, and then molded. Pre-compounding can be carried out in known equipment For example, after pre-drying the polyester composition (e.g., for four hours at 120éC), a single screw extruder can be fed with a dry blend of the ingredients, the screw employed having a long transition section to ensure proper melting. Alternatively, a twi n-screw extruder with intermeshing co-rotating screws can be fed with polyester and other components at the feed port and reinforcing fibers (and other additives) can optionally be fed downstream. In either case, a generally suitable melt temperature will be 230éC to 300éC. The pre-compounded components can be extruded and cut up into molding compounds such as granules, pellets, and the like by standard techniques. The granules or pellets can then be molded in any known equipment used for molding thermoplastic compositions, such as a Newbury orvan Dorn type injection-molding machine with cylinder temperatures at 230éC to 280éC, and mold temperatures at 55éC to 95éC. The granules and pellets can also be extruded into films or sheets. The articles formed by molding or extrusion of the thermoplastic polyester compositions possess an excellent balance of flame retardance, ductility, tensile strength and flexural modulus.

The thermoplastic compositions comprising PBT manufactured from the di(C₁₋₃)alkyl terephthalic ester residue, specifically the DMT residue, can have excellent flame retardancy when molded into either thick or thin articles. One set of test conditions commonly accepted and used as a standard for flame retardancy is set forth in Underwriters Laboratories, Inc. Bulletin 94, which prescribes specific conditions under which the self-extinguishing characteristics of a material are rated. A 0.8 mm thick molded article comprising the thermoplastic polyester composition has a UL-94 flammability rating of V0 or V1.

An article molded from the thermoplastic compositions comprising PBT manufactured from the di(C₁₋₃)alkyl terephthalic ester residue, specifically the DMT residue, can have a melt volume ratio (MV R) of 10 to 70 cm³/10 minute, more specifically 15 to 50 cm³/10 minute, measured in accordance with ISO11443 at 250lC and 2.16 kg.

An article molded from the thermoplastic compositions comprising PBT manufactured from the di(C₁₋₃)alkyl terephthalic ester residue, specifically the DMT residue having from more than 0 to less than 4.2 wt% of a residual component selected from dimethyl isophthalate, cyclohexanedimethanol, diethylene glycol, triethylene glycol, and combinations thereof, can have a tensile stress at break of at least 15 MPa, specifically at least 30 MPa, and more specifically at least 35 MPa, as measured in accordance with ASTM D 638 on molded samples having a thickness of 3.2 mm.

An article molded from the thermoplastic compositions comprising PBT manufactured from the di(C₁₋₃)alkyl terephthalic ester residue, specifically the DMT residue having from more than 0 to less than 4.2 wt %?)of a residual component selected from dimethyl isophthalate, cyclohexanedimethanol, diethylene glycol, triethylene glycol, and combinations thereof, can have a flexural modulus of from 1,700 to 4,500 MPa, more specifically 2,000 to 4,000 MPa, measured in accordance with ASTM 790.

In a specific embodiment, an article molded from the thermoplastic compositions comprising PBT manufactured from the di(C₁₋₃)alkyl terephthalic ester residue, specifically the DMT residue having from more than 0 to less than 4.2 wt.% of a residual component selected from dimethyl isophthalate, cyclohexanedimethanol, diethylene glycol, triethylene glycol, and combinations thereof, can have a tensile elongation at break of 0 to 169%, specifically at least 1% to 169%, at least 4% to 169%, or at least 10% to 169%, as measured in accordance with ASTM D638 on molded samples having a thickness of 3.2 mm.

Still further in an embodiment, an article molded from the thermoplastic compositions comprising PBT manufactured from the di(C₁₋₃)alkyl terephthalic ester residue, specifically the DMT residue having from more than 0 to less than 3.0 wt% of the residual component, , a polycarbonate, and an impact modifier has a heat deflection temperature from 87· C to 102· C when measured at 0.455 M Pa in accordance with ASTM method D648.

One or more of the foregoi ng properti es can be achieved by a thermoplastic polyester composition that consists essentially of the PBT derived from a DMT residue the DMT residue having from more than 0 to less than 4.2 wt.% of the residual component, a polycarbonate, and an impact modifier, and one or more additives selected from a mold release agent, an antioxidant, a thermal stabilizer, and a UV stabilizer. Further, one or more of the foregoing properties can be achieved by a thermoplastic polyester composition that consists of the PBT derived from a DMT residue having from more than 0 to less than 4.2 wt.% of the residual component, a polycarbonate, and an impact modifier one or more additives selected from a mold release agent, an antioxidant, a thermal stabilizer, and a UV stabilizer.

Also disclosed are extruded, shaped, or molded articles comprising the PBT manufactured from the di(C₁₋₃)alkyl terephthalic ester residue, specifically the DMT residue, such as electric and electronic parts, including, for example, connectors, fans used in electronic devices such as computers, circuit breakers, lamp holders, fusers, power distribution box, enclosures, housings, and power plugs. A method of forming an article comprises extruding, calendaring, molding, or injection molding the thermoplastic polyester composition to form the article. A specific article is a housing, in particular a housing for an electrical connector wherein at least one surface comprises the impact-modified composition.

Advantageously, our invention provides previously unavailable benefits. It provides thermoplastic PBT compositions derived at least in part from a scrap or recycle polyester, yet desirable properties, including melting temperature, crystallization temperature, and heat deflection temperature that are the same as or similar to compositi ons containing PBT derived from virgin DMT.

The invention is further illustrated by the following non-limiting exampl es.

### EXAMPLES

The materials shown in Table 1 were used in the Examples.

**Table 1.**

| Component | Chemical description | Source |
|---|---|---|
| 1,4-Butanediol | Monomer | BASF |
| Cyclohexane di methanol | Co-monomer (impurity) | Eastman |
| Dimethyl isophthalate | Co-monomer (impurity) | Fisher scientific |
| Dimethyl terephthalate | Monomer | Invista |
| Tetra(isopropyl) titanate | Catalyst | DuPont |
| Triethylene glycol | Co-monomer (impurity) | Fisher Scientific |
| Diethylene glycol | Co-monomer (impurity) | Fisher Scientific |
| Poly(butylene terephthalate) | PBT from virgin DMT | Sabic Innovative Plastics |
| Hindered phenol stabilizer | Antioxidant | C hemtura |
| Hydrocarbon wax release | Mold release | A stor |
| Brominated polycarbonate | Flame retardant polymer | Sabic Innovative Plastics |
| Polycarbonate | Polymer | Sabic Innovative Plastics |
| Antimony oxi de concentrate | Flame retardant | Industri al Plastics |
| Low density polyethylene | Mold release | Exxon Mobil |
| SAN encapsulated PTFE intermediate | Anti-drip agent | Sabic Innovative Plastics |
| Zinc phosphate | Quencher | Halox |
| Pentaerythritol beta-lauryl thiopropionate | Release agent | Chemtura |
| Anhydrous monosodi um phosphate | Quencher | Innophos |
| MBS | Methacrylate-butadiene-styrene impact modifier | Dow Advanced Chemicals |
| 2-(2-hydroxy-5-T-octylphenyl)-benzotriazole | UV stabilizer | A merichem |

### Property Testing Procedures

The melting point, Tₘ, and the crystallization temperature, T_{c}, of the polymers were measured by DSC with a 20 °C/min heating rate. Tₘ was measured on the second heating cycle.

Molecular weight data was measured by gel permeation chromatography using polystyrene standards with a 2000 D cutoff.

Melt volume rate (MVR) of the polymers (dried for 2 hr at 120l C prior to measurement) was measured accordi ng to ISO 1133 method with a dwelling time of 300 s a 0.0825 inch (2.1 mm) orifice at 250 °C with an applied load of 2.16 kg.

Meltviscosity (MV) of the polymerwas measured by ISO11443. A multi-point method is used to generate a melt viscosity curve as a function of shear rate.

Intri nsi c viscosity (IV) data was obtained for the polymer.

Color data for the polymer was acqui red by diffuse reflectance on a Gretag Macbeth Color-Eye7000A with D65 illumination, 10é observer, CIE L*, a*, b*, specular included, UV included, large lens position, large aperture.

Flexural properties were measured using the ASTM D790 method using bar di mensi ons of 127 x 12.7 x 3.2 mm, a support span of 50.8 mm, and a test speed of 1.27 mm/min.

Heat Deflection Temperature was tested on five bars having the di mensi ons 127 x 12.7 x 3.2 mm using ASTM method D648 that uses a loading stress of 0.455 or 1.82 MPa with no prior annealing.

Notched Izod impact testing was done on 76.2 x 12.7 x 3.2 mm bars using A ST M method D256. Bars were notched prior to oven aging; samples were tested at room temperature (23 °C).

U n-notched Izod impact testing was done on 76.2 x 12.7 x 3.2 mm bars using ASTM method D4812. Samples were tested at room temperature (23 °C).

Biaxial impact testing, sometimes referred to as instrumented impact testing, was done as per ASTM D3763 using 101.6 x 3.2 mm molded discs with a test velocity of 3.3 m/s. The total energy absorbed by the sample is reported as J. Testing was done at room temperature (23 °C) on as molded samples.

Mold Shrinkage was tested using 101.6 x 3.2 mm molded discs as per an internal method. Both parallel and perpendicular shrinkage measurements were made.

Tensile modul us and tensile elongation at break were tested on injection molded Type 1 bars with a gage length of 50 mm at room temperature with a crosshead speed of 50 mm/mi n using ASTM D638.

### PBT Polymerization Process

The polymerization reactions in Examples 1-9 were carried out in a 40CV Helicone reactor. The helicone reactor had a capacity of 200 L and was equipped with a special design of twin opposing helical blades with 270 degree twist; constructed of 316 SS with 16 g polish finish. The blade speed could be varied from 1 to 65 rpm. The agitators were connected to a Constant Torque Inverter Duty Motor, which operates at 230/460 VAC, 3 PH, and 60 Hz. These agitators provided excellent surface area for the polymer melt in order to build molecular weight. The helicone was also designed with an overhead condenser to condense the vapors in the transesterification and polymerization stages. The reactor had a conical design with twin rotating blades with close clearance to the reactor walls. This configuration allowed for efficient mixing of high viscosity polymer melts. The 40 CV Helicone reactor was preheated to 170 °C and loaded with all materials. For all batches 45.5 kg of 1,4-butanediol, 65.9 kg of dimethyl terephthalate, and 53.7 mL of tetra(isopropyl) titanate were added. Once the materials were added and the reactor closed, the temperature was ramped from 170°C to 227°C at 2.5 °C/min. During this stage, the 1,4-butanediol reacted with the dimethyl terephthalate and liberated methanol. Both methanol and excess 1,4-butanediol were collected by means of an overhead condenser with cooling water set to 35 °C. Once the rate of overhead collection slowed considerably, vacuum was slowly applied at a target reduction of 33 mbar/min (3.3 kPa/min) while ramping the temperature from 227 °C to 250 °C. The reactor was held at these conditions throughout the polymerization while the molecular weight of the polymer. Additional 1,4-butanediol was collected using the overhead condenser with cooling water at 55 °C. Once the torque in the reactor measured 15 amps, the agitator motor slowed and vacuum seal was broken. Once the agitator stopped, the polymer was discharged from the reactor. The polymer made in this reactor was ground after cooling using a conventional grinder with a mesh screen.

PBT was prepared in Example 10 on an industrial scale through process steps consisting of 2 main steps. First, the polyethyl ene terephthalate is depolymerized by reaction with ethylene glycol in a process known as glycolysis. The reaction product is then contacted with 1,4-butanediol under conditions suitable for polymerization.

### Dimethyl Terephthalate Compositions

To make the impurity-containing dimethyl terephthalate residue compositions, the amount of di methyl terephthalate was kept constant and one or more of the following impurities were added in the appropriate ratio: di methyl isophthalate, cyclohexane di methanol, diethylene glycol, tri ethyl ene glycol.

### Preparation of Stabilized PBT Compositions

Stabilized PBT was prepared in Examples 11-20 by dry mixing 99.74 weight % (wt.%) PBT from Examples 1-10, 0.06wt.% hindered phenol stabilizer, and 0.2 wt.% hydrocarbon wax release in a paint shaker for approximately 4 min. The mixture was then extruded on a 27 mm Entek Mega Twin Screw Extruder (with a maximum capacity of 34 kg/hr) having 5 feeders and a vacuum vented mixing screw. The feed temperature was 204 éC. The extrusion temperature was maintained between 204-260 eC. The screw speed was 450 rpm. The extrudate was cooled through a water bath prior to pelletizing. Test parts were injection molded on a van Dorn molding machi ne. The pellets were dried for 4 hr at 121 éC in a forced air-circulating oven prior to injection molding. Parts for testing were molded with a barrel temperature set to 260 lC and the mold temperature set to 66l C. ASTM parts were made for all tests. Cycle time was approximately 35 s.

### Preparation of Flame Retardant PBT Compositions

A flame retardant PBT with a very high PBT content was prepared in Examples 21-30 by dry mixing 64.46 wt.% PBT from Examples 1-10, 26.0 wt% brominated polycarbonate, 2.0 wt% polycarbonate, 6.1 wt.% antimony oxide concentrate, 1.0 wt% low density polyethylene, 0.06 wt.% hindered phenol stabilizer, 0.08 wt.% SAN encapsulated PT FE -intermediate, and 0.3 wt% zinc phosphate in a paint shaker for approximately 4 min. The preparation procedure was otherwise identical to that of Examples 11-20.

### Preparation of Impact Modified Polycarbonate/ PBT Compositions

An impact modified polycarbonate / PBT blend using a high PBT content was prepared in Examples 31-40 by dry mixing 66.32 wt.% PBT from Examples 1-10, 15.0 wt.% polycarbonate, 18.0 wt.% MBS rubber, 0.25 wt.% 2-(2'hydroxy-5-t-octylphenyl)-benzotriazole, 0.08 wt.% hindered phenol stabilizer, 0.05 wt.% pentaerythritol beta-lauryl thiopropionate, and 0.3 wt.% anhydrous monosodium phosphate in a paint shaker for approximately 4 min. The preparation procedure was otherwise identical to that of Examples 11-20.

### EXAMPLES 1-10

### Examples 1-4.

The purpose of Example 1 was to prepare PBT from dimethyl terephthalate residue compositions containing dimethyl isophthalate. More specifically, the dimethyl terephthalate composition was made using the procedure described above, except that the following impurity was added: 3.0 wt.% dimethyl isophthalate.

PBT was prepared from dimethyl terephthalate contai ni ng 3.0 wt.% dimethyl isophthalate on a pilot scale with the dimethyl terephthalate being composed of 63.9 kg dimethyl terephthalate and 2.0 kg dimethyl isophthalate. The process steps and conditions were otherwise identical to those described above. The results for Example 1 are shown in Table 2.

The purpose of Example 2 was to prepare PBT from dimethyl terephthalate residue compositions containing cyclohexane dimethanol. More specifically, the dimethyl terephthalate composition was made using the procedure described above, except that the following impurities were added: 1.5 wt.% cyclohexane dimethanol.

PBT was prepared from dimethyl terephthalate containing 1.5 wt.% cyclohexane dimethanol on a pilot scale with the dimethyl terephthalate being composed of 64.9 kg of dimethyl terephthalate and 1.0 kg of cyclohexane dimethanol. The process steps and conditions were otherwise identical to those described above. The results for Example 2 are shown in Table 2.

The purpose of Example 3 was to prepare PBT from dimethyl terephthalate residue compositions containing diethylene glycol and triethylene glycol. More specifically, the dimethyl terephthalate composition was made using the procedure described above, except that the following impurities were added: 0.5 wt.% diethylene glycol and 0.1 wt.% tri ethylene glycol.

PBT was prepared from dimethyl terephthalate containing 0.5 wt.% diethyl ene glycol and 0.1 wt.% tri ethyl ene glycol on a pilot scale with the dimethyl terephthalate being composed of 65.5 kg of dimethyl terephthalate, 330 g of diethylene glycol and 66 g of triethylene glycol. The process steps and conditions were otherwise identical to those described above. The results for Example 3 are shown in Table 2.

The purpose of Example4 was to prepare PBT from dimethyl terephthalate residue compositions containing dimethyl isophthalate, cyclohexane di methanol, diethylene glycol, and triethylene glycol. More specifically, the dimethyl terephthalate composition was made using the procedure described above, except that the following impurities were added: 3 wt% dimethyl isophthalate, 1.5 wt.% cyclohexane dimethanol, 0.5 wt.% diethylene glycol, and 0.1 wt.% tri ethylene glycol.

PBT was prepared from dimethyl terephthalate containi ng 3 wt% dimethyl isophthalate, 1.5 wt% cyclohexane dimethanol, 0.5 wt.% diethylene glycol, and 0.1 wt.% triethylene glycol on a pilot scale with the dimethyl terephthalate being composed of 62.7 kg of dimethyl terephthalate, 2.0 kg of dimethyl isophthalate, 1.0 kg of cyclohexane dimethanol, 330 g of diethylene glycol and 66 g of triethylene glycol. The process steps and conditions were otherwise identical to those described above. Results for Example 4 are in Table 2.

### Comparative Examples 5-9.

The purpose of Comparative Examples 5-9 was to prepare PBT from dimethyl terephthalate using the procedure described above, but with no impurities added in the dimethyl terephthalate.

PBT was prepared on the pilot scale using 65.0 kg of dimethyl terephthalate. The process steps and testing conditions were otherwise identical to those described above for Examples 1-4. The results for Comparative Examples 5-9 are shown in Table 2.

### Comparative Example 10.

The purpose of Comparative Example 10 was to compare PBT made from polyethylene terephthalate. This material is used for comparison purposes with Examples 1-4. While this material is not made from dimethyl terephthalate, this PBT contains a known amount of impurities that are ordinarily present in recycled polyethylene terephthalate sources. The results for Comparative Example 10 are shown in Table 2.

Table 2 shows the measured properti es for Examples 1-4 and Comparative Examples 5-10. Amounts are shown in weight percent.

**Table 2.**

| Ex. | DMI | CHDM | DEG | TEG | DMT Residual Composition Residue Level | Onset Tₘ (°C) | Onset T_{c} (°C) | L* | a* | b* |
|---|---|---|---|---|---|---|---|---|---|---|
| 1 | 3.0 | 0 | 0 | 0 | 3.0 | 220.6 | 178.0 | 83.91 | 0.849 | 8.41 |
| 2 | 0 | 1.5 | 0 | 0 | 1.5 | 222.1 | 177.8 | 84.31 | 0.702 | 7.93 |
| 3 | 0 | 0 | 0.5 | 0.1 | 0.6 | 224.2 | 177.8 | 85.87 | 0.157 | 5.47 |
| 4 | 3.0 | 1.5 | 0.5 | 0.1 | 5.1 | 216.3 | 168.4 | 86.48 | 0.48 | 6.62 |
| 5 | 0 | 0 | 0 | 0 | 0 | 225.4 | 180.5 | 85.98 | 1.113 | 9.35 |
| 6 | 0 | 0 | 0 | 0 | 0 | 223.9 | 186.9 | 86.69 | 0.509 | 6.44 |
| 7 | 0 | 0 | 0 | 0 | 0 | 225.5 | 177.6 | 89.56 | -0.039 | 3.77 |
| 8 | 0 | 0 | 0 | 0 | 0 | 225.8 | 176.3 | 87.54 | -0.094 | 3.64 |
| 9 | 0 | 0 | 0 | 0 | 0 | 225.4 | 182.2 | 87.36 | -0.201 | 4.15 |
| 10 | n/d | n/d | n/d | n/d | 4.1 | 215.7 | 174.9 | 73.64 | -2.025 | 2.77 |

The results in Table 2 show that the use of only certain dimethyl terepthalate compositions containing specific levels of impurities produced polybutylene terepthalate having suitable properties.

PBT polymers made in Comparative Examples 5-9 from dimethyl terephthalate were each tested twice. This allowed establishment of total process variation, including testing. This baseline was then used to detect any significant deviation in polymer properti es of the materials made in Examples 1-4 from the Comparative Examples. A significant deviation was a property measurement more than 2 standard deviations from the baseline properties established from the Comparative Examples.

The color of polymers made from recycled materials is often an issue. The L*, a*, b* measurements for the produced polymers are given in Table 3. Comparative Example 10 gives results that are typical of PBT made from recycled polyethylene terephthalate sources by glycolysis. The color measurements for Examples 1-4 are all within the normal process variation. Presence of the co-monomers in the amounts used in Examples 1-4 does not have a significant adverse effect on the color of the produced PBT.

**Table 3. Comparison of polymer color of Examples 1-4 to baseline PBT.**

| Ex. | Co-monomers | L* | a* | b* |
|---|---|---|---|---|
| 1 | 3% DMI | 84 | 0.8 | 8.4 |
| 2 | 1.5% CHDM | 84 | 0.7 | 7.9 |
| 3 | 0.5% DEG, 0.1% TEG | 86 | 0.2 | 5.5 |
| 4 | 3% DMI, 1.5% CHDM, 0.5% DEG, 0.1% TEG | 86 | 0.5 | 6.6 |
| 5-9 | None | 84 - 91 | -0.9 - 1.3 | 0.9 - 9.3 |
| 10 | 4.1% Total | 74 | -2.0 | 2.8 |

In the pilot scale helicone reactor, the molecular weight of the polymer was not tightly controlled. Any dependence of melt viscosity, melt volume rate, and intrinsic viscosity on the impurities in dimethyl terephthalate was insignificant compared to the molecular weight variations inherent to the process.

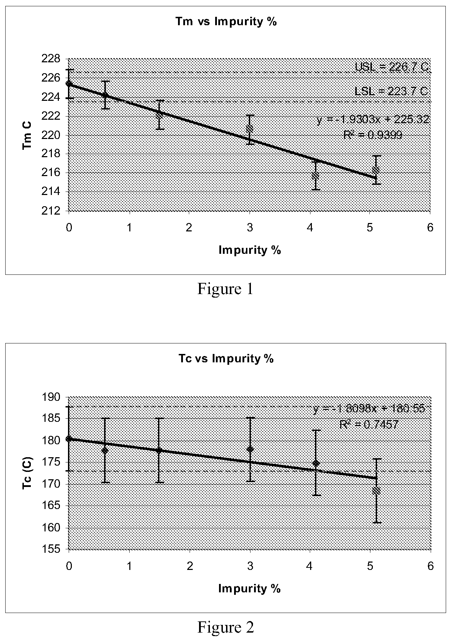

The melting point (Tₘ) and the crystallization temperature (T_{c}) of the polymers made in Examples 1-10 as a function of dimethyl terephthalate impurity are shown in Figure 1 and Figure 2, respectively. The points at 0 wt.% impurity correspond to the average of the 5 batches of PBT made from virgin dimethyl terephthalate in Examples 5-9, each measured twice. The standard deviation for the process was calculated from the PBT made from virgin dimethyl terephthalate. The error bars on the points are given as 2 process standard deviations on each side. The specifications shown in the Figures correspond to 2 standard deviations on either side of the standard PBT. These are included to see if a significant change in the measured property occurs due to the impurities in the dimethyl terephthalate. Points marked with squaresindicate measurements that are significantly different than the baseline established using Examples 5-9. Both Tₘ and T_{c} show a dependence upon the amount of impurities in the dimethyl terephthalate used to make PBT. The effect of impurities on Tₘ is much stronger than their effect on T_{c} relative to the process standard deviation and has a linear relationship between the Tₘ and the impurity level. From the thermal data, it does not appear that the type of iimpurity has an impact, only the total amount of impurity in the dimethyl terephthalate. Surprisingly, even very low impurity levels result in significant changes to the Tₘ of the polymer. Surprisingly, if the total amount of impurities present in the dimethyl terephthalate is more than only 0.8 wt.%, the PBT will have a significantly different melting point. To ensure that the PBT made from recycled dimethyl terephthalate has equivalent melting point properties, the recycled dimethyl terephthalate must contain less than 0.8 wt.% of the impurities.

### EXAMPLES 11-20

### Examples 11-14.

The purpose of Example 11 was to prepare stabilized PBT from PBT made from dimethyl terephthalate residue compositions containing dimethyl isophthalate. More specifically, the stabilized PBT composition was prepared using PBT made from dimethyl terephthalate residue compositions containing 3.0 wt.% dimethyl isophthalate from Example 1, hindered phenol stabilizer, and hydrocarbon wax release according to the procedure described above. The testing procedures were identical to those described above. The results for Example 11 are shown in Table 4.

The purpose of Example 12 was to prepare stabilized PBT from PBT made from dimethyl terephthalate residue compositions containing cyclohexane dimethanol. More specifically, the stabilized PBT composition was prepared using PBT made from dimethyl terephthalate residue compositions containing 1.5 wt.% cyclohexane dimethanol from Example 2, hindered phenol stabilizer, and hydrocarbon wax release according to the procedure described above. The process steps and conditions were otherwise identical to those described above. The results for Example 12 are shown in Table 4.

The purpose of Example 13 was to prepare stabilized PBT from PBT made from dimethyl terephthalate residue compositions containing diethylene glycol and triethylene glycol. More specifically, the stabilized PBT composition was prepared using PBT prepared using dimethyl terephthalate residue compositions containing 0.5 wt.% diethylene glycol and 0.1 wt.% tri ethyl ene glycol from Example 3, hindered phenol stabilizer, and hydrocarbon wax release accordi ng to the procedure descri bed above. The process steps and conditions were otherwise identical to those described above. The results for Example 13 are shown in Table 4.

The purpose of Example 14 was to prepare stabilized PBT from PBT made from dimethyl terephthalate residue compositions containing dimethyl isophthalate, cyclohexane dimethanol, diethylene glycol, and triethylene glycol. More specifically, the stabilized PBT composition was prepared using PBT prepared from dimethyl terephthalate residue compositions containing 3 wt.% dimethyl isophthalate, 1.5 wt.% cyclohexane dimethanol, 0.5 wt.% diethylene glycol and 0.1 wt.% triethylene glycol from Example 4, hindered phenol stabilizer, and hydrocarbon wax release. The process steps and conditions were otherwise identical to those described above. The results for Example 14 are in Table 4. Comparative Examples 15-19.

The purpose of Comparative Examples 15-19 was to establish baseline process variation for the production of stabilized PBT compositions made from virgin dimethyl terephthalate, a material that contained substantially less of the impurities that were found in the dimethyl terephthalate residue compositions. The stabilized PBT was prepared from PBT made from the dimethyl terephthalate residues without added impurities in Comparative Examples 5-9, hindered phenol stabilizer, and hydrocarbon wax release according to the procedure described above. The process steps and conditions were otherwise identical to those described above. The results for Comparative Examples 15-19 are shown in Table 4.

### Comparative Example 20.

The purpose of Comparative Example 20 was to compare stabilized PBT compositions comprising PBT made from recycled polyethylene terephthalate. This material is used for comparison purposes with Examples 11-14. While this material is not made from dimethyl terephthalate, this stabilized PBT contains a known amount of impurities that are ordinarily present in recycled polyethylene terephthalate sources. The stabilized PBT was prepared using PBT made from polyethylene terephthalate from Comparative Example 10, hindered phenol stabilizer, and hydrocarbon wax release according to the procedure described above. The process steps and conditions were otherwise identical to those described above. The results for Comparative Example 20 are shown in Table 4. Results for Examples 11-20.

Table 4 below shows the results for stabilized PBT made in Examples 11-20.

**Table 4.**

| | |
|---|---|
| FM -flexural modulus | NIIS- Notched Izod impact strength, |
| UIIS - Unnotched Izod impact strength | E - modulus of elasticity |
| MSPII - Mold shri nkage in the parallel direction, | e_{y} - elongation at break |
| MSPpn - Mold shrinkage in the perpendicular direction | |

Stabilized PBT polymers made in Comparative Examples 15-19 containing PBT produced from dimethyl terephthalate were each extruded, molded, and tested twice. This allowed establishment of total process variation, including testing. This baseline was then used to detect any significant deviation in polymer properties of the materials made in Examples 11-14 from the Comparative Examples 15-19. A significant deviation was a property measurement more than 2 standard deviations from the baseline properties established from the Comparative Examples 15-19.

Measurements of flexural modulus, notched Izod impact strength, unnotched Izod impact strength, and the modulus of elasticity for Examples 11-20 are all within the normal process variation. The presence of the co-monomers in the amounts used in Examples 1-4 does not have a significant adverse effect on these properties of the stabilized PBT compositions.

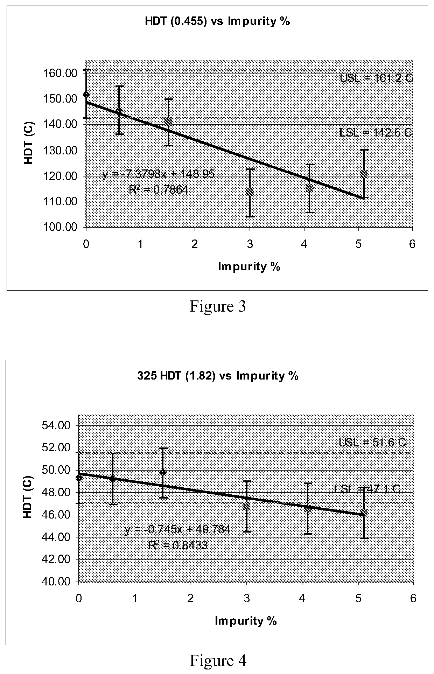

The heat deflection temperature measurements of the stabilized PBT polymers with a loading stress of 0.455 MPa and 1.82 MPa are shown in Figure 3 and Figure 4, respectively. Points marked with squares indicate measurements that are significantly different than the baseline established using Examples 15-19. The heat deflection temperature measurements for Examples 11-14 show a significant reduction when impurities are present in the dimethyl terephthalate used to make the stabilized PBT. Surprisingly, the presence of the co-monomers in the amounts used in Examples 1-4 does have a significant adverse effect on the heat deflection temperature of the produced stabilized PBT. This is believed to be due to incorporation of the impurities as co-monomers in the polymer

| Ex. | DMT Residual Level | FM (MPa) | 0.455 MPa HDT (°C) | 1.82 MPa HDT (°C) | NIIS (J/m) | UIIS (J/m) | Total Energy (J) | MS PII (%) | MS Ppn (%) | E (MPa) | e_{y} (%) |
|---|---|---|---|---|---|---|---|---|---|---|---|
| 11 | 3.0 | 2520 | 113.5 | 46.8 | 49.6 | 2130 | 56.1 | 1.97 | 1.52 | 2770 | 190.8 |
| 12 | 1.5 | 2510 | 141 | 49.8 | 49 | 2100 | 51.7 | 1.96 | 1.59 | 2690 | 246.4 |
| 13 | 0.6 | 2540 | 145.7 | 49.3 | 49.5 | 2130 | 64.8 | 2.06 | 2.08 | 2770 | 96.1 |
| 14 | 5.1 | 2470 | 120.9 | 46.2 | 51 | 2090 | 45.9 | 1.94 | 1.85 | 2690 | 283.6 |
| 15 | 0 | 2590 | 155 | 49.9 | 49.5 | 1750 | 29.4 | 2.27 | 2.23 | 2850 | 6.9 |
| 16 | 0 | 2570 | 151.8 | 49.1 | 28.3 | 2040 | 12.8 | 2.08 | 2.15 | 2810 | 68.8 |
| 17 | 0 | 2480 | 146.8 | 47.7 | 37.8 | 1830 | 41.5 | 2.17 | 2.2 | 2720 | 123.5 |
| 18 | 0 | 2580 | 153.3 | 52.1 | 27.2 | 572 | 4.54 | 2.16 | 2.15 | 2810 | 5.3 |
| 19 | 0 | 2490 | 148.5 | 48.9 | 26.4 | 2140 | 77.8 | 2.11 | 2.18 | 2650 | 89.8 |
| 20 | 4.1 | 2540 | 115.2 | 46.6 | 51.9 | 2140 | 4.503 | 2.02 | 2.04 | 2730 | 276 |

backbone resulting in a decrease in the crystallinity of the polymer. Surprisingly, even low impurity levels result in significant changes to the heat deflection temperature of the polymer. If the total impurities present in the di methyl terephthalate are more than only 0.9 wt.%, the stabilized PBT may have a significantly different heat deflection temperature. To ensure that the stabilized PBT made from recycled dimethyl terephthalate has an equivalent heat deflection temperature, the recycled dimethyl terephthalate must contain less than 0.9 wt.% of impurities.

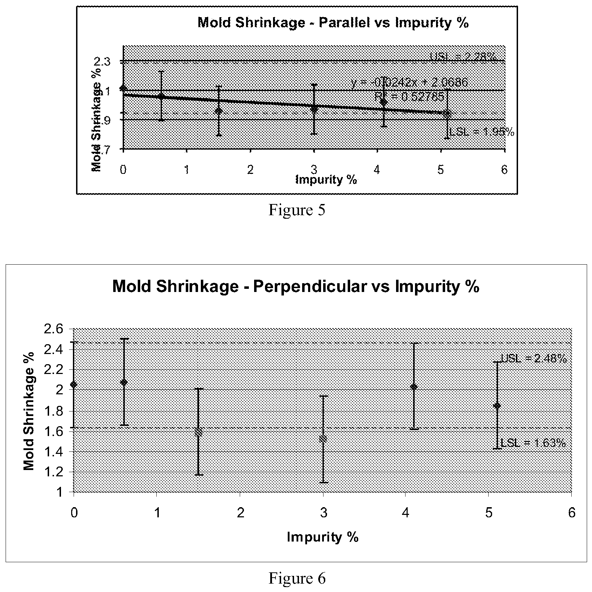

The mold shri nkage measurements in the parallel direction and in the perpendicular direction of the stabilized PBT compositions are shown in Figure 5 and Figure 6, respectively. Both the mold shrinkage measurements in the parallel direction and in the perpendicular direction show a dependence upon the amount of impurities in the dimethyl terephthalate used to make PBT. Surprisingly, even low impurity levels result in significant changes to the mold shrinkage of the polymer. This is also believed to be due to incorporation of the impurities as co-monomers in the polymer backbone resulting in a decrease in the crystallinity of the polymer. If the total i mpuriti es present in the di methyl terephthalate are more than only 5.1 wt.% the stabilized PBT may have significantly different mold shrinkage in the parallel direction and if the total impurities present in the dimethyl terephthalate are more than only 1.5 wt.% the stabilized PBT may have significantly different mold shrinkage in the perpendicular direction. To ensure that the stabilized PBT made from recycled dimethyl terephthalate has equivalent mold shrinkage, the recycled dimethyl terephthalate must contain less than 1.5 wt.% of impurities.

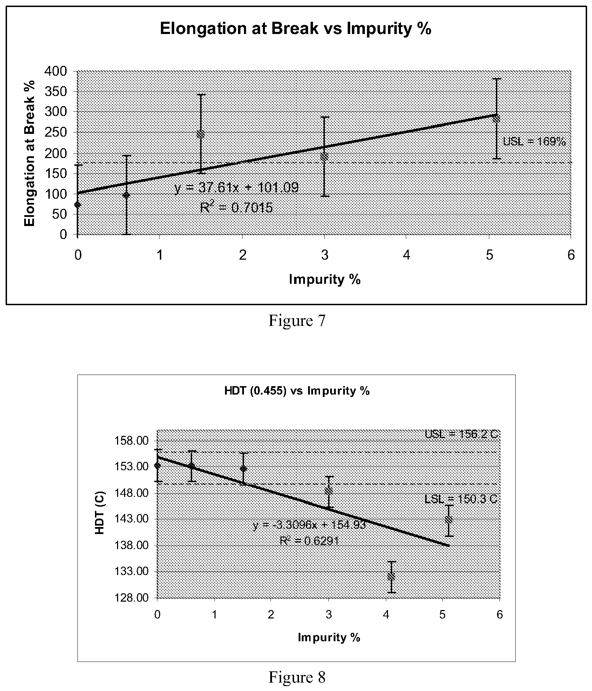

The tensile elongation at break measurements of the stabilized PBT compositi ons are shown in Figure 7. The tensile elongation at break measurements show a dependence upon the amount of impurities in the dimethyl terephthalate used to make PBT. Surprisingly, even low impurity levels result in significant changes to the elongation at break of the polymer. If the total i mpuriti es present in the di methyl terephthalate are more than only 1.5 wt.%, the stabilized PBT may have significantly different elongation at break. To ensure that the stabilized PBT made from recycled dimethyl terephthalate has equivalent elongation at break, the recycled dimethyl terephthalate must contain less than 1.5 wt.% of i mpuriti es.

To ensure that the stabilized PBT made from di methyl terephthalate residual compositions has equivalent heat deflection properties, tensile elongation at break, and mold shrinkage in the perpendicular and parallel directions, as well as flexural modulus, notched Izod impact strength, un-notched Izod impact strength, mold shrinkage in both the parallel and perpendicular directions, the modulus of elasticity, and elongation at break, the recycled dimethyl terephthalate must contain less than 0.9 wt.% of impurities.

### EXAMPLES 21-30

### Examples 21-24

The purpose of Example 21 was to prepare a flame retardant PBT with a very high PBT content made from dimethyl terephthalate residue compositions. More specifically, the flame retardant was prepared using PBT made from dimethyl terephthalate residue compositions containing 3.0 wt.% dimethyl isophthalate from Example 1, brominated polycarbonate, polycarbonate, hindered phenol stabilizer, antimony oxide concentrate, SAN encapsulated PTFE-intermediate, low density polyethylene, and zinc phosphate according to the procedure described above. The process steps and conditions were otherwise identical to those described above. The results for Example 21 are shown in Table 5.

The purpose of Example 22 was to prepare a flame retardant PBT with a very high PBT content from PBT made from dimethyl terephthalate residue compositions containing cyclohexane di methanol. More specifically, the flame retardant was prepared using PBT made from dimethyl terephthalate residue compositions containing 1.5 wt.% cyclohexane dimethanol from Example 2, brominated polycarbonate, polycarbonate, hi ndered phenol stabilizer, antimony oxide concentrate, SAN encapsulated PTFE-intermediate, low density polyethylene, and zinc phosphate according to the procedure described above. The process steps and conditions were otherwise identical to those described above. The results for Example 22 are shown in Table 5.

The purpose of Example 23 was to prepare a flame retardant PBT with a very high PBT content from PBT made from dimethyl terephthalate residue compositions containing diethylene glycol and triethylene glycol. More specifically, the flame retardant was prepared using PBT made from dimethyl terephthalate residue compositions containing 0.5 wt.% diethylene glycol and 0.1 wt% triethylene glycol from Example 3, brominated polycarbonate, polycarbonate, hindered phenol stabilizer, antimony oxide concentrate, SAN encapsulated PTFE-intermediate, low density polyethylene, and zinc phosphate according to the procedure described above. The process steps and conditions were otherwise identical to those described above. The results for Example 23 are shown in Table 5.

The purpose of Example 24 was to prepare a flame retardant with a very high PBT content from PBT made from dimethyl terephthalate residue compositions containing dimethyl isophthalate, cyclohexane dimethanol, diethyl ene glycol, and triethylene glycol. More specifically, the flame retardant was prepared using PBT made from dimethyl terephthalate residue compositions containing 3.0 wt.% dimethyl isophthalate, 1.5 wt.% cyclohexane dimethanol, 0.5 wt.% diethylene glycol and 0.1 wt.% triethylene glycol from Example 4, brominated polycarbonate, polycarbonate, hindered phenol stabilizer, antimony oxide concentrate, SAN encapsulated PTFE-intermediate, low density polyethylene, and zinc phosphate according to the procedure described above. The process steps and conditions were otherwise identical to those described above. Results for Example 24 are in Table 4. Comparative Examples 25-29

The purpose of Comparative Examples 25-29 was to establish baseline process variation for the production of a flame retardant with a very high PBT content produced from the polymer made from virgin dimethyl terephthalate, a material that contained substantially less of the impurities that were found in the dimethyl terephthalate residue compositions. This material is used for comparison with Examples 21-24. The flame retardant PBT was prepared using PBT made from dimethyl terephthalate from Comparative Examples 5-9, brominated polycarbonate, polycarbonate, hindered phenol stabilizer, antimony oxide concentrate, SAN encapsulated PTFE-intermediate, low density polyethylene, and zinc phosphate according to the procedure described above. PBT is made from virgin dimethyl terephthalate using the same processing and testing conditions as used in Examples 21-24. The results for Comparative Examples 25-29 are shown in Table 5. Comparative Example 30

The purpose of Example 30 was to compare a flame retardant with a very high PBT content made from PBT made from polyethylene terephthalate. This material is used for comparison purposes with Examples 21-24. While this material is not made from di methyl terephthalate, this flame retardant with a very high PBT content contains a known amount of impurities that are ordinarily present in recycled polyethylene terephthalate sources. The flame retardant PBT was prepared using polyethylene terephthalate from comparative Example 10, brominated polycarbonate, polycarbonate, hindered phenol stabilizer, antimony oxide concentrate, SAN encapsulated PTFE-intermediate, low density polyethylene, and zinc phosphate according to the procedure described above. The testing procedures were identical to those described above. The results for Comparative Example 30 are shown in Table 5.

### Results for Examples 21-30

Table 5 shows the results for stabilized PBT made in Examples 21-30.

**Table 5.**

| FM -flexural modulus | | | | | | NIIS- Notched Izod impact strength, | | | | | |
|---|---|---|---|---|---|---|---|---|---|---|---|
| Ex. | DMT Residual Composition Level | FM (MPa) | 0.455 MPa HDT (°C) | 1.82 MPa HDT (°C) | NIIS (J/m) | UIIS (J/m) | Total Energy (J) | MSPII (%) | MSPpn (%) | E (MPa) | e_{y} (%) |
| 21 | 3.0 | 2710 | 148.3 | 66.1 | 53.4 | 1470 | 23.4 | 1.6 | 1.6 | 2920 | 11.1 |
| 22 | 1.5 | 2690 | 152.7 | 68.1 | 49.6 | 1290 | 8.86 | 1.55 | 1.55 | 2870 | 4.9 |
| 23 | 0.6 | 2720 | 153.2 | 75.2 | 51 | 1490 | 51.6 | 1.58 | 1.58 | 2920 | 8.7 |
| 24 | 5.1 | 2640 | 142.8 | 60.4 | 54.8 | 1630 | 7.66 | 1.49 | 1.43 | 2830 | 6.6 |
| 25 | 0 | 2710 | 154.5 | 69.8 | 51.9 | 1520 | 22.1 | 1.62 | 1.61 | 2910 | 5.4 |
| 26 | 0 | 2670 | 153.4 | 68.8 | 30.4 | 1810 | 29.3 | 1.81 | 1.85 | 2830 | 10.3 |
| 27 | 0 | 2680 | 154.4 | 70.2 | 33.1 | 1470 | 36.7 | 1.82 | 1.84 | 2830 | 9.5 |
| 28 | 0 | 2700 | 154.9 | 70.1 | 30.8 | 1920 | 40.5 | 1.88 | 1.9 | 2850 | 14 |
| 29 | 0 | 2660 | 152.4 | 65.1 | 30.8 | 1770 | 54 | 1.81 | 1.83 | 2820 | 15.2 |
| 30 | 4.1 | 2710 | 132 | 61.2 | 55.9 | 2130 | 63.5 | 1.38 | 1.37 | 2900 | 24.9 |

| | | | | | | | | | | | |
|---|---|---|---|---|---|---|---|---|---|---|---|
| UIIS - Unnotched Izod impact strength MSPII - Mold shrinkage in the parallel direction, MSPpn - Mold shrinkage in the perpendicular direction E - modulus of elasticity e_{y} - elongation at break | | | | | | | | | | | |

Flame retardant PBT compositions of Comparative Examples 25-29 made from PBT from dimethyl terephthalate were each extruded, molded, and tested twice. This allowed establishment of total process variation, including testing. This baseline was then used to detect any significant deviation in properties of the materials made in

Examples 21-24 from the Comparative Examples 25-29. A significant deviation was a property measurement more than 2 standard deviations from the baseline properties established from the Comparative Examples 25-29. The points at 0 wt.% impurity correspond to the average of the 5 batches using PBT made from virgin dimethyl terephthalate in Examples 25-29, each measured twice. Points marked with squares indicate measurements that are significantly different than the baseline established using Examples 25-29.

Measurements of the flexural modulus, notched Izod impact strength, un-notched Izod impact strength, mold shrinkage in the parallel and perpendicular directions, modulus of elasticity, and tensile elongation at break of the produced flame retardant with a very high PBT content do not show any correlation that depends on the amount of impurities present. Presence of the co-monomers in the amounts used in Examples 1-4 does not have a significant adverse effect on these properties of the produced flame retardant with a very high PBT content.

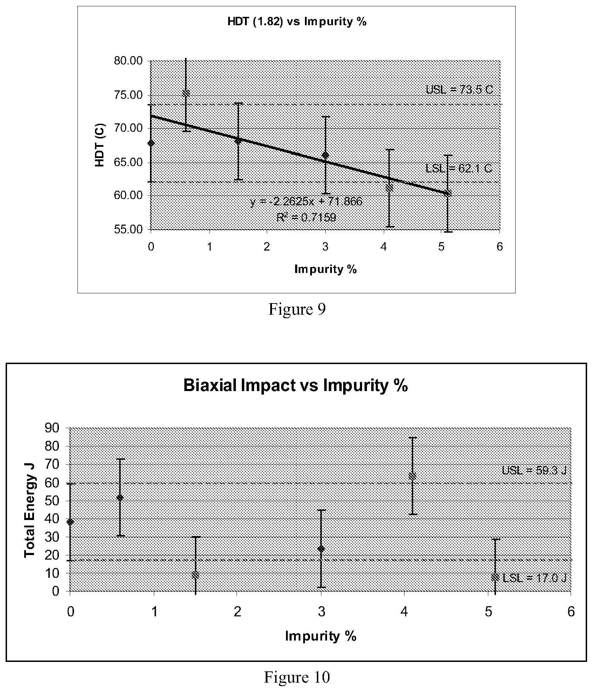

The heat deflection temperature measurements of the produced flame retardant with a very high PBT content with a loading stress of 0.455 MPa and 1.82 MPa are given in Figure 8 and Figure 9, respectively. The heat deflection temperature measurements for Examples 21-24 show a significant reduction when impurities are present in the dimethyl terephthalate used to make the flame retardant with a very high PBT content. Surprisingly, the presence of the co-monomers in the amounts used in Examples 1-4 does have a significant adverse effect on the heat deflection temperature of the produced flame retardant with a very high PBT content. This is believed to be due to incorporation of the impurities as co-monomers in the PBT backbone resulting in a decrease in the crystallinity of the polymer. Surprisingly, even low impurity levels result in significant changes to the heat deflection temperature of the flame retardant with a very high PBT content If the total impurities present in the dimethyl terephthalate are more than only 1.4 wt.%, the flame retardant with a very high PBT content may have a significantly different heat deflection temperature. To ensure that the flame retardant with a very high PBT content made from recycled di methyl terephthalate has an equivalent heat deflection temperature, the recycled dimethyl terephthalate must contain less than 1.4 wt.% of impurities.

The total energy measurements from the biaxial impact test of the produced flame retardant with a very high PBT content are given in Figure 10. The total energy measurements for Examples 21-24 do not have a correlation to the amount of impurities present in the dimethyl terephthalate used to make the PBT. However, three of the exampl es do have total energy measurements from the biaxial impact test that are significantly different than the Comparative Examples. If the total impurities present in the di methyl terephthalate are more than only 1.5 wt.%, the flame retardant with a very high PBT content may have a significantly different total energy measured by the biaxial impact test. To ensure that the flame retardant with a very high PBT content made from recycled dimethyl terephthalate has equivalent properties, the recycled dimethyl terephthalate must contain less than 1.5 wt.% of impurities

### EXAMPLES 31-40

### Examples 31-34

The purpose of Example 31 was to prepare an impact modified polycarbonate/ PBT blend using a high PBT content from PBT made from dimethyl terephthalate residue compositions containing dimethyl isophthalate. More specifically, the impact modified polycarbonate/ PBT blend was prepared using PBT made from dimethyl terephthalate residue compositions containing 3.0 wt.% dimethyl isophthalate from Example 1, polycarbonate, MBS, 2-(2'hydroxy-5-t-octylphenyl)-benzotriazole, hindered phenol stabilizer, pentaerythritol beta-lauryl thiopropionate, and anhydrous monosodium phosphate according to the procedure described above. The process steps and conditions were otherwise identical to those described above. The results for Example 31 are shown in Table 5.

The purpose of Example 32 was to prepare an impact modified polycarbonate/ PBT blend using a high PBT content from PBT made from dimethyl terephthalate residue compositions containing cyclohexane dimethanol. More specifically, the impact modified polycarbonate / PBT blend was prepared using PBT made from dimethyl terephthalate residue compositions containing 1.5 wt.% cyclohexane dimethanol from Example2, polycarbonate, MBS, 2-(2'hydroxy-5-t-octylphenyl)-benzotriazole, hindered phenol stabilizer, pentaerythritol beta-lauryl thiopropionate, and anhydrous monosodium phosphate according to the procedure described above. The process steps and conditions were otherwise identical to those described above. The results for Example 32 are in Table 6.

The purpose of Example 33 was to prepare an impact modified polycarbonate/ PBT blend using a high PBT content from PBT made from dimethyl terephthalate residue compositions containing diethylene glycol and triethylene glycol. More specifically, the impact modified polycarbonate / PBT blend was prepared using PBT made from dimethyl terephthalate residue compositions containing 0.5 wt.% diethylene glycol and 0.1 wt.% triethyleneglycol from Example3, polycarbonate, MBS rubber, 2-(2'hydroxy-5-t-octylphenyl)-benzotriazole, hindered phenol stabilizer, pentaerythritol beta-lauryl thiopropionate, and anhydrous monosodium phosphate according to the procedure described above. The process steps and conditions were otherwise identical to those described above. The results for Example 33 are shown in Table 6.

The purpose of Example 34 was to prepare an impact modified polycarbonate/PBT blend using a high PBT content from PBT made from dimethyl terephthalate residue compositions containing dimethyl isophthalate, cyclohexane dimethanol, diethylene glycol, and triethylene glycol. More specifically, the impact modified polycarbonate/ PBT blend was prepared using PBT made from dimethyl terephthalate residue compositions containing 3.0 wt.% dimethyl isophthalate, 1.5 wt.% cyclohexane dimethanol, 0.5 wt.% diethylene glycol and 0.1 wt.% triethylene glycol from Example 4, polycarbonate, MBS rubber, 2-(2'hydroxy-5-t-octylphenyl)-benzotriazole, hindered phenol stabilizer, pentaerythritol beta-lauryl thiopropionate, and anhydrous monosodium phosphate according to the procedure described above. The process steps and conditions were otherwise identical to those described above. The results for Example 34 are \ in Table 6. Comparative Examples 35-39

The purpose of Comparative Examples 35-39 was to establish baseline process variation for the production of an impact modified with a polycarbonate / PBT blend using a high PBT content produced from the polymer made from virgin dimethyl terephthalate, a material that contained substantially less of the impurities that were found in the dimethyl terephthalate residue compositions. The impact modified with a polycarbonate/PBT blend was prepared using a high PBT content from PBT made from dimethyl terephthalate from Comparative Examples 5-9, polycarbonate, MBS rubber, 2-(2'hydroxy-5-t-octylphenyl)-benzotriazole, hindered phenol stabilizer, pentaerythritol beta-lauryl thiopropionate, and anhydrous monosodium phosphate according to the procedure described above. The process steps and conditions were otherwise identical to those described above. This material is used for comparison purposes with Examples 31-34. The results for Comparative Examples 35-39 are shown in Table 6.

### Comparative E xampl e 40

The purpose of Comparative Example 40 was to compare an impact modified polycarbonate/PBT blend using a high PBT content from PBT made from polyethylene terephthalate. This material is used for comparison purposes with Examples 31-34. While this material is not made from dimethyl terephthalate, this impact modified with a polycarbonate/ PBT blend using a high PBT content contains a known amount of impurities that are ordinarily present in recycled polyethyl ene terephthalate sources. The impact modified with a polycarbonate / PBT blend was prepared using a high PBT content from PBT made from polyethylene terephthalate from Comparative Example 10, polycarbonate, MBS rubber, 2-(2'hydroxy-5-t-octylphenyl)-benzotriazole, hindered phenol stabilizer, pentaerythritol beta-lauryl thiopropionate, and anhydrous monosodium phosphate according to the procedure descri bed above. The testing procedures were identical to those descri bed above. The results for Comparative Example 40 are shown in Table 6. Results for Examples 31-40

Table 6 shows the properties for an impact modified polycarbonate / PBT blend using a high PBT content of Examples 31-40.

**Table 6.**

| Ex. | DMT Residual Composition Level | FM (MPa) | 0.455 MPa HDT (°C) | 1.82 MPa (°C) | NIIS (J/m) | UIIS (J/m) | Total Energy (J) | MSPII (%) | MSPpn (%) | E (MPa) | e_{y} (%) |
|---|---|---|---|---|---|---|---|---|---|---|---|
| 31 | 3.0 | 1840 | 93.6 | 52.1 | 786 | 2040 | 52.7 | 1.44 | 1.43 | 1790 | 147.6 |
| 32 | 1.5 | 1830 | 96.4 | 53.3 | 755 | 1940 | 53.3 | 1.44 | 1.42 | 1750 | 71.6 |
| 33 | 0.6 | 1800 | 95.6 | 53.3 | 791 | 1970 | 56.5 | 1.45 | 1.44 | 1780 | 96.2 |
| 34 | 5.1 | 1780 | 82.5 | 51.4 | 858 | 1870 | 54.6 | 1.45 | 1.47 | 1710 | 155.1 |
| 35 | 0 | 1860 | 100.6 | 55.2 | 810 | 2040 | 54.6 | 1.52 | 1.5 | 1810 | 87.2 |
| 36 | 0 | 1770 | 95.2 | 52.8 | 838 | 1920 | 56.4 | 1.43 | 1.44 | 1780 | 178.9 |
| 37 | 0 | 1770 | 95.9 | 52.8 | 872 | 2050 | 56.2 | 1.51 | 1.53 | 1750 | 235.3 |
| 38 | 0 | 1740 | 93.8 | 52.7 | 823 | 1920 | 53.1 | 1.46 | 1.53 | 1730 | 103.4 |
| 39 | 0 | 1760 | 91.2 | 53.9 | 836 | 2040 | 56.3 | 1.45 | 1.45 | 1780 | 126.9 |
| 40 | 4.1 | 1760 | 87.8 | 51.9 | 842 | 2000 | 55.6 | 1.37 | 1.35 | 1660 | 236.2 |

| | | | | | | | | | | | |
|---|---|---|---|---|---|---|---|---|---|---|---|
| FM -flexural modulus UIIS - Unnotched Izod impact strength MSPll - Mold shrinkage in the parallel direction, MSPpn - Mold shrinkage in the perpendicular direction NIIS- Notched Izod impact strength E - modulus of elasticity e_{y} - elongation at break | | | | | | | | | | | |

Impact modified polycarbonate/ PBT polymers made using a high PBT content made in Comparative Examples 35-39 made from PBT from dimethyl terephthalate were each extruded, molded, and tested twice. This allowed establishment of total process variation, including testing. This baseline was then used to detect any significant deviation in properties of the materials made in Examples 31-34 from the Comparative Examples 35-39. A significant deviation was a property measurement more than 2 standard deviations from the baseline properties established from the Comparative Examples 35-39. The points at 0 wt.% impurity correspond to the average of the 5 batches using PBT made from virgin dimethyl terephthalate in Examples 35-39, each measured twice. Points marked with squares indicate measurements that are significantly different than the baseline established using Examples 35-39.

Measurements of the flexural modulus, notched Izod impact strength, un-notched Izod impact strength, total energy measured by the biaxial impact test, mold shrinkage in the parallel and perpendicular directions, modulus of elasticity, and tensile elongation at break of the produced impact modified with a polycarbonate / PBT blend using a high PBT content do not show any correlation that depends on the amount of impurities present. Presence of the co-monomers in the amounts used in Examples 1-4 does not have a significant adverse effect on these properties of the produced impact modified with a polycarbonate/ PBT blend using a high PBT content.

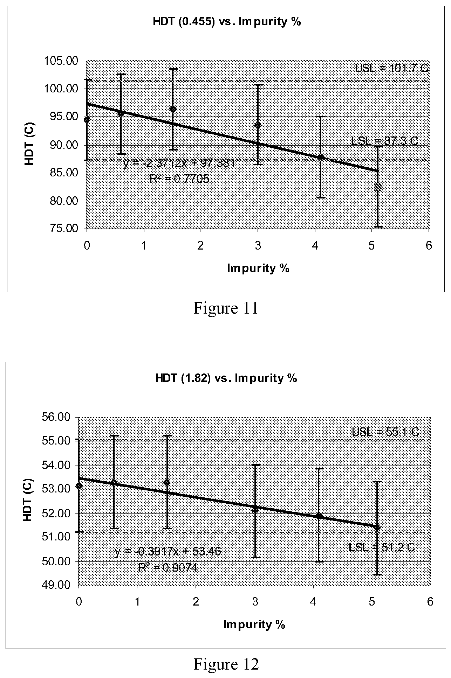

The heat deflection temperature measurements of the produced impact modified with a polycarbonate / PBT blend using a high PBT content with a loading stress of 0.455 MPa and 1.82 MPa are given in Figure 11 and Figure 12, respectively. The heat deflection temperature measurements for Examples 31-34 show a significant reduction when impurities are present in the dimethyl terephthalate used to make the impact modified with a polycarbonate/ PBT blend using a high PBT content. Surprisingly, the presence of the co-monomers in the amounts used in Examples 1-4 does have a significant adverse effect on the heat deflection temperature of the produced impact modified with a polycarbonate / PBT blend using a high PBT content. This is believed to be due to incorporation of the impurities as co-monomers in the PBT backbone resulting in a decrease in the crystallinity of the polymer. Surprisingly, even low impurity levels result in significant changes to the heat deflection temperature of the impact modified with a polycarbonate/ PBT blend using a high PBT content. If the total impurities present in the dimethyl terephthalate are more than only 4.2 wt.%, the impact modified with a polycarbonate/ PBT blend using a high PBT content may have a significantly different heat deflection temperature. To ensure that the impact modified with a polycarbonate / PBT blend using a high PBT content made from recycled dimethyl terephthalate has an equivalent heat deflection temperature, the recycled dimethyl terephthalate must contain less than 4.2 wt.% of impurities.

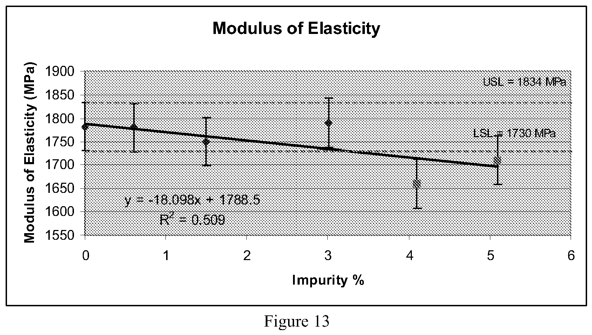

The modulus of elasticity measurements of the produced impact modified with a polycarbonate / PBT blend using a high PBT content are given in Figure 13. The modulus of elasticity measurements for Examples 31-34 show a significant reduction when impurities are present in the dimethyl terephthalate used to make the impact modified with a polycarbonate / PBT blend using a high PBT content. Surprisingly, the presence of the co-monomers in the amounts used in Examples 1-4 does have a significant adverse effect on the modulus of elasticity of the produced impact modified with a polycarbonate / PBT blend using a high PBT content. If the total impurities present in the dimethyl terephthalate are more than only 3.2 wt.%, the impact modified with a polycarbonate / PBT blend using a high PBT content may have a significantly different total energy measured by the biaxial impact test. To ensure that the impact modified with a polycarbonate / PBT blend using a high PBT content made from recycled dimethyl terephthalate has an equivalent modulus of elasticity, the recycled di methyl terephthalate must contain less than 3.2 wt.% of i mpuriti es. To ensure that the impact modified with a polycarbonate / PBT blend using a high PBT content made from recycled dimethyl terephthalate has equivalent properties, the recycled dimethyl terephthalate must contain less than 3.2 wt.% of impurities.

## Claims

1. A poly(butyleneterephthalate) composition comprising
a poly(butyleneterephthalate) copolymer reaction product of a dimethyl terephthalate residual composition with 1,4-butanediol,
wherein the dimethyl terephthalate residual composition comprises
a. dimethyl terepthalate derived from a terephthalic-containing polyester homopolymer, terephthalic-containing polyester copolymer, or a combination thereof; and
b. from more than 0 to less than 4.2 wt.% of reaction residues of the residual component selected from dimethyl isophthalate, cyclohexane dimethanol, diethylene glycol, triethylene glycol, and combinations thereof.

2. A poly(butyleneterephthalate) composition comprising
(a) a polybutylene terepthalate copolymer reaction product of 1,4-butanediol and the dimethyl terephthalate residual composition comprising from more than 0 to less than 4.2 wt.% of a residual component selected from dimethyl isophthalate, cyclohexane dimethanol, diethylene glycol, triethylene glycol, and combinations thereof of claim 1;
(b) a polycarbonate; and
(c) an impact modifier;
wherein the polybutylene terephthalate composition has a heat deflection temperature ranging from 87 éC to 102 éC, when measured at 0.455 MPa in accordance with ASTM method D648.

3. The impact-modified poly(butyleneterephthalate) composition of Claim 2 comprising
from 30 to 90 wt.% of the polybutylene terepthalate copolymer reaction product;
from 10 to 80 wt.% of the polycarbonate;
from more than 0 to 25 wt.% of the impact modifier; and further comprisi ng:
from more than 0 to 2 wt.% of an antioxidant;
from more than 0 to 2 wt.% of a mold release agent;
from more than 0 to 2 wt.% of a quencher; and
from more than 0 to 2 wt.% of an ultraviolet light stabilizer,
wherein the polybutylene terephthalate composition has a heat deflection temperature ranging from 87 °C to 102 °C, when measured at 0.455 MPa in accordance with ASTM method D648.

4. The poly(butylene terephthalate) composition of Claim2 or 3, wherein the polycarbonate is a bisphenol A polycarbonate.

5. The poly(butylene terephthalate) composition of Claim 2 or 3, wherein the impact modifier is a rubbery material selected from of (a) methacrylate butadiene styrene rubbers, (b) acrylate rubbers, (c) acrylonitrile-styrene-acrylate rubbers, (d) high rubber graft acrylonitrile-butadiene-styrenes, (e) acrylate-olefin copolymers, (f) polyolefin modifiers, (g) silicone-acrylic modifiers, and combinations thereof.

6. The poly(butylene terephthalate) composition of Claim 2 or 3, wherein the impact modifier is a methacrylate-butadiene-styrene rubbers.

7. The poly(butyleneterephthalate) composition of any of Claims 2-6, further comprising a flame retardant selected from sulfonated salts, brominated compounds, antimony flame retardants, phosphorus-containing flame retardants, nitrogen-containing flame retardants, phosphinate salts, diphosphinate salts, and combinations thereof.

8. The poly(butyleneterephthalate) composition of any of Claims 2-7, further comprising an additive selected from antioxidants, thermal stabilizers, radiation stabilizers, and ultraviolet light absorbing additives, mold release agents, plasticizers, quenchers, lubricants, antistatic agents, dyes, pigments, laser marking additives, processing aids, pigments, dyes, and combinations thereof.

9. The poly(butyleneterephthalate) composition of any of Claims 2-8, wherein the composition further comprises an antioxidant, a mold release agent, a quencher, and an ultraviolet light stabilizer.

10. A method of manufacture of the impact-modified, flame retardant poly(butylene terephthalate) composition of any of Claims 2-9, comprising melt blending the components.

11. A n article comprising the poly(butylene terephthalate) of any of Claims 2-9.

12. The article of Claim 11, wherein the article is in the form of a housing having at least one surface comprising the composition of any of Claims 2-9.

13. The article of Claim 12, wherein the housing is a housing of an electrical connector.

14. A method of manufacture of an article, comprising molding, extruding, shaping, or calendering the composition of any of Claims 2-9.

## Patentansprüche

1. Eine Poly(butylenterephthalat)-Zusammensetzung, umfassend
ein Poly(butylenterephthalat)-Copolymer-Reaktionsprodukt aus einer Dimethylterephthalat-Residualzusammensetzung mit 1,4-Butandiol,
wobei die Dimethylterephthalat-Residualzusammensetzung umfasst
a. Dimethylterepthalat, abgeleitet von einem terephthalhaltigen Polyesterhomopolymer, einem terephthalhaltigen Polyestercopolymer oder einer Kombination davon; und
b. von mehr als 0 bis weniger als 4,2 Gew.-% der Reaktionsresiduen der Residualkomponente, die aus Dimethylisophthalat, Cyclohexandimethanol, Diethylenglykol, Triethylenglykol und Kombinationen davon ausgewählt sind.

2. Eine Poly(butylenterephthalat)-Zusammensetzung, umfassend
(a) ein Polybutylenterepthalat-Copolymer-Reaktionsprodukt aus 1,4-Butandiol und der Dimethylterephthalat-Residualzusammensetzung, die mehr als 0 bis weniger als 4,2 Gew.-% einer Residualkomponente umfasst, welche aus Dimethylisophthalat, Cyclohexandimethanol, Diethylenglykol, Triethylenglykol und Kombinationen davon nach Anspruch 1 ausgewählt ist;
(b) ein Polycarbonat; und
(c) einen Impakt-Modifikator;
wobei die Polybutylenterephthalatzusammensetzung eine Wärmeformbeständigkeitstemperatur im Bereich von 87 °C bis 102 °C aufweist, gemessen bei 0,455 MPa gemäß ASTM-Verfahren D648.

3. Die schlagmodifizierte Poly(butylenterephthalat)-Zusammensetzung nach Anspruch 2, umfassend
von 30 bis 90 Gew.-% des Polybutylenterepthalat-Copolymer-Reaktionsprodukts;
von 10 bis 80 Gew.-% des Polycarbonats;
von mehr als 0 bis 25 Gew.-% des Impakt-Modifikators; und weiterhin aufweist:
von mehr als 0 bis 2 Gew.-% eines Antioxidans;
von mehr als 0 bis 2 Gew.-% eines Formtrennmittels;
von mehr als 0 bis 2 Gew.-% eines Abschreckmittels; und
von mehr als 0 bis 2 Gew.-% eines ultravioletten Lichtstabilisators,
wobei die Polybutylenterephthalatzusammensetzung eine Wärmeformbeständigkeitstemperatur im Bereich von 87 °C bis 102 °C aufweist, wenn bei 0,455 MPa gemäß ASTM-Methode D648 gemessen wird.

4. Die Poly(butylenterephthalat)-Zusammensetzung nach Anspruch 2 oder 3, worin das Polycarbonat ein Bisphenol A-Polycarbonat ist.

5. Die Poly(butylenterephthalat)-Zusammensetzung nach Anspruch 2 oder 3, worin der Impakt-Modifikator ein gummiartiges Material ist, ausgewählt aus (a) Methacrylat-Butadien-Styrol-Kautschuken, (b) Acrylatkautschuken, (c) Acrylnitril-Styrol-Acrylat-Kautschuken, (d) Hochgummi-Pfropfacrylnitril-Butadien-Styrolen, (e) Acrylat-Olefin-Copolymeren, (f) Polyolefin-Modifikatoren, (g) Silikon-Acryl-Modifikatoren und Kombinationen davon.

6. Die Poly(butylenterephthalat)-Zusammensetzung nach Anspruch 2 oder 3, worin der Impakt-Modifikator ein Methacrylat-Butadien-Styrol-Kautschuk ist.

7. Die Poly(butylenterephthalat)-Zusammensetzung nach einem der Ansprüche 2-6, ferner umfassend ein flammwidrig machendes Mittel, ausgewählt aus sulfonierten Salzen, bromierten Verbindungen, Antimonflammschutzmitteln, phosphorhaltigen Flammschutzmitteln, stickstoffhaltigen Flammschutzmitteln, Phosphinatsalzen, Diphosphinatsalzen und Kombinationen davon.

8. Die Poly(butylenterephthalat)-Zusammensetzung nach einem der Ansprüche 2-7, ferner umfassend ein Additiv, ausgewählt aus Antioxidantien, Wärmestabilisatoren, Strahlenschutzmitteln und UV-Licht absorbierenden Additiven, Formtrennmitteln, Weichmachern, Abschreckmitteln, Schmiermitteln, Antistatikmitteln, Farbstoffen, Pigmenten, Lasermarkierungsadditiven, Verarbeitungshilfsmitteln, Pigmenten, Farbstoffen und Kombinationen davon.

9. Die Poly(butylenterephthalat)-Zusammensetzung nach einem der Ansprüche 2-8, wobei die Zusammensetzung ferner ein Antioxidans, ein Formtrennmittel, ein Abschreckmittel und einen UV-Lichtstabilisator umfasst.

10. Ein Verfahren zur Herstellung der impakt-modifizierten, flammhemmenden Poly(butylenterephthalat)-Zusammensetzung nach einem der Ansprüche 2-9, umfassend das Mischen der Komponenten durch Schmelzen.

11. Ein Artikel, umfassend das Poly(butylenterephthalat) nach einem der Ansprüche 2-9.

12. Der Artikel nach Anspruch 11, worin der Artikel die Form eines Gehäuses mit mindestens einer Oberfläche hat, die die Zusammensetzung eines der Ansprüche 2-9 aufweist.

13. Der Artikel nach Anspruch 12, worin das Gehäuse ein Gehäuse eines elektrischen Verbinders ist.

14. Ein Verfahren zur Herstellung eines Artikels, umfassend das Formen, Extrudieren, Formgeben oder Kalandrieren der Zusammensetzung nach einem der Ansprüche 2-9.

## Revendications

1. Composition de polybutylène téréphtalate, comprenant un produit de réaction de copolymère de polybutylène téréphtalate à partir d'une composition résiduelle de diméthyl téréphtalate avec du 1,4-butanediol,
dans laquelle la composition résiduelle de diméthyl téréphtalate comprend
a. du diméthyl téréphtalate dérivé d'un homopolymère de polyester contenant du téréphtalique, d'un copolymère de polyester contenant du téréphtalique, ou d'une combinaison de ceux-ci ; et
b. de plus de 0 à moins de 4,2 % en poids de résidus de réaction du composant résiduel choisi parmi le diméthyl isophtalate, le cyclohexane diméthanol, le diéthylène glycol, le triéthylène glycol et les combinaisons de ceux-ci.

2. Composition de polybutylène téréphtalate, comprenant
(a)un produit de réaction de copolymère de polybutylène téréphtalate à partir de 1,4-butanediol et de la composition résiduelle de diméthyl téréphtalate comprenant plus de 0 à moins de 4,2 % en poids d'un composant résiduel choisi parmi le diméthyl isophalate, le cyclohexane diméthanol, le diéthylène glycol, le triéthylène glycol et les combinaisons de ceux-ci selon la revendication 1 ;
(b)un polycarbonate ; et
(c)un agent de modification de la résistance aux chocs ;
dans laquelle la composition de polybutylène téréphtalate a une température de fléchissement sous charge allant de 87 °C à 102 °C, mesurée à 0,455 MPa d'après la méthode ASTM D648.

3. Composition de polybutylène téréphtalate ayant une résistance aux chocs modifiée selon la revendication 2, comprenant
de 30 à 90 % en poids du produit de réaction de copolymère de polybutylène téréphtalate ;
de 10 à 80 % en poids de polycarbonate ;
de plus de 0 à 25 % en poids de l'agent de modification de la résistance aux chocs ; et comprenant en outre :
de plus de 0 à 2 % en poids d'un antioxydant ;
de plus de 0 à 2 % en poids d'un agent de démoulage ;
de plus de 0 à 2 % en poids d'un agent de désactivation ; et
de plus de 0 à 2 % en poids d'un stabilisateur vis-à-vis de la lumière ultraviolette,
dans laquelle la composition de polybutylène téréphtalate a une température de fléchissement sous charge allant de 87 °C à 102 °C, mesurée à 0,455 MPa d'après la méthode ASTM D648.

4. Composition de polybutylène téréphtalate selon la revendication 2 ou 3, dans laquelle le polycarbonate est un polycarbonate de bisphénol A.

5. Composition de polybutylène téréphtalate selon la revendication 2 ou 3, dans laquelle l'agent de modification de la résistance aux chocs est un matériau caoutchouteux choisi parmi (a) des caoutchoucs de méthacrylate butadiène styrène, (b) des caoutchoucs d'acrylate, (c) des caoutchoucs d'acrylonitrile - styrène - acrylate, (d) des styrènes d'acrylonitrile - butadiène greffés hautement caoutchouteux, (e) des copolymères d'acrylate - oléfine, (f) des agents de modification de polyoléfine, (g) des agents de modification de silicone - acrylique, et des combinaisons de ceux-ci.

6. Composition de polybutylène téréphtalate selon la revendication 2 ou 3, dans laquelle l'agent de modification de la résistance aux chocs est un caoutchouc de méthacrylate - butadiène - styrène.

7. Composition de polybutylène téréphtalate selon l'une quelconque des revendications 2 à 6, comprenant en outre un agent ignifuge choisi parmi les sels sulfonatés, les composés bromés, les agents ignifuges à l'antimoine, les agents ignifuges contenant du phosphore, les agents ignifuges contenant de l'azote, les sels de phosphinate, les sels de diphosphinate et des combinaisons de ceux-ci.

8. Composition de polybutylène téréphtalate selon l'une quelconque des revendications 2 à 7, comprenant en outre un additif choisi parmi les antioxydants, les thermostabilisants, les stabilisateurs vis-à-vis du rayonnement, et les additifs absorbant la lumière ultraviolette, les agents de démoulage, les plastifiants, les agents de désactivation, les lubrifiants, les agents antistatiques, les colorants, les pigments, les additifs de marquage laser, les auxiliaires de transformation, les pigments, les colorants, et des combinaisons de ceux-ci.

9. Composition de polybutylène téréphtalate selon l'une quelconque des revendications 2 à 8, dans laquelle la composition comprend en outre un antioxydant, un agent de démoulage, un agent de désactivation et un stabilisateur vis-à-vis de la lumière ultraviolette.

10. Méthode de fabrication de la composition de polybutylène téréphtalate ignifuge dont la résistance aux chocs a été modifiée selon l'une quelconque des revendications 2 à 9, comprenant le mélange en fusion des composants.

11. Article comprenant le polybutylène téréphtalate selon l'une quelconque des revendications 2 à 9.

12. Article selon la revendication 11, dans lequel l'article est sous la forme d'un boîtier ayant au moins une surface comprenant la composition selon l'une quelconque des revendications 2 à 9.

13. Article selon la revendication 12, dans lequel le boîtier est un boîtier d'un connecteur électrique.

14. Méthode de fabrication d'un article, comprenant le moulage, l'extrusion, la mise en forme ou le calandrage de la composition selon l'une quelconque des revendications 2 à 9.
